# EUROPÄISCHE PATENTSCHRIFT

(11) **EP 1 526 876 B1**
(45) Veröffentlichungstag und Bekanntmachung des Hinweises auf die Patenterteilung: **06.12.2006**
(21) Anmeldenummer: 03790893.6
(22) Anmeldetag: 08.08.2003
(51) Int. Cl.: A61L 9/01, A61L 9/02, A61L 9/03

(54) **DUFTABGABESYSTEM**
FRAGRANCE RELEASE SYSTEM
SYSTEME DE LIBERATION DE SUBSTANCES ODORIFERANTES

(30) Priorität: 09.08.2002 DE 10237066; 29.01.2003 DE 10303352
(43) Veröffentlichungstag der Anmeldung: 04.05.2005
(73) Patentinhaber: Henkel Kommanditgesellschaft auf Aktien, 40589 Düsseldorf (DE)
(72) Erfinder: BARTHEL, Wolfgang, 40764 Langenfeld (DE); CANAVOIU-OPRITESCU, Ion, 40591 Düsseldorf (DE); REIMANN, Matthias, 40593 Düsseldorf (DE); KESSLER, Arnd, 40789 Monheim (DE); MÜLHAUSEN, Hans-Georg, 40597 Düsseldorf (DE)
(86) Internationale Anmeldenummer: PCT/EP2003/008853
(87) Internationale Veröffentlichungsnummer: WO 2004/020004

(56) Entgegenhaltungen:
- WO-A-88/01503
- WO-A-91/00744
- US-A- 5 460 787
- US-A1- 2002 058 595
- US-B1- 6 213 409

## Beschreibung

Die Erfindung betrifft ein Duftabgabesystem mit einem Behälter und einer Vielzahl von im Aufnahmeraum des Behälters aufgenommenen Partikeln zur Desodorierung und/oder Beduftung von offenen bzw. geschlossenen Räumen, insbesondere Geschirrspülmaschinen, Textilmaschinen, Wäschetrockner, wobei die Partikel ein vorzugsweise polymeres Trägermaterial sowie wenigstens einen Duftstoff aufweisen, wobei der Behälter eine Mehrzahl von Öffnungen aufweist, durch welche eine Emittierung der Duftstoffe der Partikel vom Aufnahmeraum nach außen möglich ist.

In geschlossenen Räumen ohne ausreichende Frischluftzufuhr treten häufig unangenehme Gerüche auf. Derartige Räume können beispielsweise die Innenräume von Geschirrspülmaschinen sein, wenn das dort befindliche Geschirr sehr stark verschmutzt ist und/oder über einen längeren Zeitraum vor dem Spülvorgang in der Spülmaschine verbleibt. Um solche Gerüche zu beseitigen oder zu verringern, sind beispielsweise für Geschirrspülmaschinen sogenannte Geschirrspülmaschinendeos bekannt. Diese Deos können auf sehr unterschiedliche Weise konfektioniert werden. Für den Verbraucher ist es dabei wünschenswert, einen Artikel zur Desodorierung von Geschirrspülmaschinen oder anderen geschlossenen Räumen zu erhalten, der bei seiner Bereitstellung einen intensiven Produktduft aufweist, welcher nicht nur eine Produktidentifikation gewährleistet, sondern gleichzeitig den Eindruck hoher Wirkstärke vermittelt und im Laufe seiner Lebensdauer eine möglichst verläßliche Freisetzung konstanter Duftstoffmengen gewährleistet. Eine Reihe verschiedener Deodorantien für Geschirrspülmaschinen sind im Stand der Technik beschrieben.

Ein gattungsgemäßes Duftabgabesystem ist aus WO 02/09779 A1 bekannt. Dieses bekannte Duftabgabesystem weist einen Behälter auf, in welchem eine Mehrzahl von kleinen mit Duftstoffen beladenen Partikeln aufgenommen sind. Der Behälter ist dabei mit einer Mehrzahl von Öffnungen versehen, deren Größe so dimensioniert ist, dass die kleinen Partikel durch die Öffnungen nicht austreten können. Andererseits sind die Öffnungen so dimensioniert, dass eine Emittierung der Duftstoffe der Partikel vom Aufnahmeraum des Behälters nach außen möglich ist. Der Behälter selbst ist bevorzugt etwa quaderförmig ausgebildet, wobei er abgerundete Ecken und einen offen- und verschließbaren Deckel aufweist. Dabei kann der Aufnahmeraum des Behälters in zwei Abschnitte unterteilt sein, in denen parfümierte Partikel angeordnet sind. Die Partikel selbst bestehen bevorzugt aus einem polymeren Trägermaterial, das mit einem Duftstoff beladen ist.

Bei dem bekannten Duftabgabesystem ist der Behälter vorzugsweise zwischen 5 und 95 % des Volumens des Aufnahmeraumes mit Partikeln befüllt, weil ein möglichst großes Partikelvolumen aber gleichwohl eine freie Beweglichkeit der Partikel erreicht werden soll.

Das bekannte Duftabgabesystem ist jedoch mit Nachteilen behaftet, die zum einen auf die Ausbildung des Behälters zurückzuführen sind, denn die beschriebene Behälterform eignet sich beispielsweise sehr schlecht zur Aufnahme in einer Geschirrspülmaschine oder dergl., da der Behälter relativ groß ist. Zum anderen ist es bei der gewählten Behälterform praktisch nicht möglich, den Aufnahmeraum des Behälters nahezu vollständig mit Partikeln zu befüllen, was jedoch wünschenswert ist, weil insbesondere bei der Anwendung innerhalb einer Geschirrspülmaschine eine Beweglichkeit der Partikel unerwünscht ist, da dann praktisch die Gesamtoberfläche aller Partikel zur Duftstoffabgabe gleichzeitig zur Verfügung steht und die Funktionsdauer des Systems begrenzt ist.

Andere Lösungen zur Desodorierung und/oder Beduftung von Spülbecken oder auch Geschirrspülmaschinen sind aus DE 100 55 193 A1 und DE 100 36 850 A1 der Anmelderin bekannt.

Duftstoffhaltige Artikel aus Ethylen/-Vinylacetat-Copolymer werden in der Druckschrift WO 91/00744 A1 offenbart. Es handelt sich dabei um Spritzgußgeformte Kunststoffplatten, die in die Geschirrspülmaschine eingehängt werden können. Da der Duftstoff vor dem Spritzgießen unter den Kunststoff gemischt, wird dieser während der Verarbeitung einer hohen thermischen Belastung ausgesetzt. Eine solche thermische Belastung führt zu einem teilweisen Verlust von Duftstoffen durch Verdunstung oder thermische Zersetzung. Derartige Kunststoffplatten zeigen ebenfalls kein über deren Gebrauchsdauer konstantes Freisetzungsprofil für die enthaltenen Duftstoffe.

Gegenstand der Druckschrift WO 85/00981 A1 ist ein Verfahren zur Imprägnierung von Kunststoffteilchen mit Duftstoffen bei niedrigen Temperaturen. Die Teilchen können unter anderem in Geschirrspülmaschinen zur Desodorierung eingesetzt werden. Diese Teilchen sollen zwischen zehn und zwanzig Spülgänge lang haltbar sein.

Auch Deodorantien für den Gebrauch in Wäschetrocknern sind aus dem Stand der Technik bekannt, so beschreibt die Druckschrift US 6,235,705 B1 ein Produkt zur Beduftung im Wäschetrockner, welches aus duftstoffhaltigen Kunststoffperlen in einem Netzbeutel besteht. Die Beduftung der Perlen geschieht während ihrer Herstellung bei erhöhter Temperatur.

Aufgabe der Erfindung ist es, ein gattungsgemäßes Duftabgabesystem so zu verbessern, dass bei möglichst geringem Raumbedarf des Behälters eine effektive Duftabgabe auch über einen längeren Zeitraum gewährleistet ist.

Weitere Aufgabe der Erfindung ist es, ein Mittel zur Desodorierung von Räumen, insbesondere von Geschirrspülmaschineninnenräumen bereitzustellen, welches ein eine erhöhte Duftfreisetzung zu Beginn des Einsatzes (Produktidentifikation, Nachweis der Wirkstärke) und eine nachfolgend verringerte, aber konstante Beduftung aufweist, wobei das Mittel lange wirksam und seiner Wirksamkeit unabhängig von Umwelt- Faktoren wie Temperatur, Feuchtigkeit oder Alkalinität ist.

Erfindungsgemäß wird die Aufgabe durch die Merkmale des Patentanspruches 1 gelöst. Die sich daran anschließenden Unteransprüche 2 bis 19 schreiben weitere Ausführungsformen.

Das erfindungsgemäße Duftabgabesystem weist einen Aufnahmeraum eines im Wesentlichen rotationssymmetrischen Behälters auf, der eine mondsichelartige Querschnittsform mit einer nach außen gewölbten Vorderwand und einer nach innen gewölbten Rückwand hat. Es beinhaltet somit einen Behälter, der eine Form aufweist, die etwa der Form eines Fliegenpilzkopfes entspricht, wobei der rotationssymmetrische Aufnahmeraum einen etwa mondsicheiartigen Querschnitt aufweist.

Es hat sich herausgestellt, dass bei dieser Behälterform ein optimales Verhältnis zwischen der Gesamtoberfläche sämtlicher Partikel im Ausgangszustand zur Gesamtoberfläche des Aufnahmeraumes aufweist, d.h. der äußeren Oberfläche der Partikel nach der ersten Anwendung, wenn der Schmelz- bzw. Erweichungspunkt der Partikel überschritten worden ist. Dieses Verhältnis ist dabei wesentlich günstiger als bei bekannten Behälterformen, es ist auch deutlich günstiger als andere Behälterformen (z.B. Kugel). Dabei ist die Oberfläche des Partikelkonglomerates ausreichend groß, um eine effektive Duftabgabe zu gewährleisten. Die Partikel bestehen dabei vorzugsweise aus solchen Materialien, wie diese insbesondere in den Patentansprüchen der vorgenannten Patentanmeldung beschrieben sind, worauf ausdrücklich Bezug genommen wird und welche zum Bestandteil der Offenbarung dieser Anmeldung gemacht werden.

Durch die Gestaltung des Duftabgabesystems sind die Partikel nicht lose im Behälter angeordnet und bilden nach der erstmaligen Anwendung nach entsprechender Erwärmung ein querschnittlich mondsichelförmiges Konglomerat, wodurch die Oberfläche der Polymerpartikel reduziert und eine längere Funktionalität des Systems ermöglicht wird. Wenn dagegen die Partikel frei beweglich im Behälter wären, wäre die Funktionsdauer des Systems nur von der Duftstoffmenge abhängig, die in jedem Partikel enthalten ist, die Anzahl der Partikel würde nur die Duftintensität beeinflussen. Durch die Reduzierung der Oberfläche der Partikel wird erreicht, dass diese nicht auf einmal ihren Duft abgeben können, die Duftintensität wird von der Partikelmenge an der Oberfläche des Konglomerates, d.h. an den Innenwandungen des Behälters im direkten Kontakt mit der Luft bestimmt. Duftstoffe aus den inneren Partikeln migrieren progressiv zur Oberfläche des Konglomerates, die inneren Partikel haben somit eine Depotfunktion für das Duftabgabesystem und ermöglichen gegenüber bekannten Systemen eine wesentlich längere Funktionsdauer.

In bevorzugter Ausgestaltung ist vorgesehen, dass die beiden Endbereiche der mondsichelartigen Querschnittsform des Aufnahmeraumes abgerundet ausgebildet sind. Der entsprechende umlaufende Randbereich des Behälters ist somit abgerundet bzw. wulstartig, so dass auch dieser Bereich vollständig mit Partikeln befüllt werden kann, wodurch der Raumbedarf des Behälters kleingehalten werden kann, was z.B. bei Einsatz in einer Geschirrspülmaschine von Bedeutung ist.

Der Behälter kann grundsätzlich auf unterschiedliche Weise hergestellt werden, er kann beispielsweise einteilg durch einen Blasformvorgang hergestellt und dann durch eine Öffnung im Behälter mit den Partikeln befüllt werden, wobei diese Öffnung nach der Befüllung anschließend verschlossen wird.

Ganz besonders bevorzugt ist jedoch vorgesehen, dass der Behälter zweiteilig ausgebildet ist, wobei der eine Teil die Rückwand und der andere Teil die Vorderwand aufweist. Die beiden Teile des Behälters können dann auf einfache Weise im Spritzgießverfahren hergestellt werden.

Um die beiden Behälterteile möglichst einfach miteinander verbinden zu können, ist bevorzugt vorgesehen, dass der die Rückwand aufweisende Teil des Behälters einen wulstartigen Randbereich aufweist, welcher mit einem stegartigen Randbereich des anderen Teiles verbunden ist.

Dabei können die beiden Teile auf unterschiedliche Weise miteinander verbunden werden, bevorzugt sind sie mittels einer Rastverbindung miteinander verbunden.

Um bei der zweiteiligen Ausbildung des Behälters des Duftabgabesystems auf einfache Weise eine weitgehend vollständige Befüllung des Behälters mit Partikeln zu gewährleisten, ist ganz besonders bevorzugt vorgesehen, dass die nach innen gewölbte Rückwand in ihrem mittleren Bereich kegelförmig nach innen gewölbt ist. In der Befüllposition bildet dann der die nach außen gewölbte Vorderwand aufweisende andere Teil zunächst die Aufnahme für die Partikel. Die benötigte Menge an Partikeln zur vollständigen Befüllung des Behälters wird in diesen Behälterteil eingefüllt, so dass der Füllstand etwas unterhalb des Behälterteilrandes verbleibt. Anschließend wird der die nach innen gewölbte Rückwand aufweisende Teil quasi als Deckel aufgelegt und eingerastet. Durch den mittleren, kegelförmig nach innen eingewölbten Bereich werden dabei die Partikel nach außen und oben verdrängt und gelangen auch in die noch nicht gefüllten Bereiche des so gebildeten Aufnahmeraumes. Die Dimensionierung ist dabei so getroffen, dass, bevor die Partikel den Rand erreichen, der wulstartige Randbereich des einen Teiles den stegartigen Randbereich des anderen Teiles erreicht, so dass die Partikel nicht herausfallen können.

In ganz besonders bevorzugter Ausgestaltung ist vorgesehen, dass der Behälter des Duftabgabesystems bzw. dessen Aufnahmeraum so dimensioniert ist, dass das Verhältnis der Gesamtoberfläche sämtlicher Partikel im Ausgangszustand (also vor ihrer erstmaligen Erwärmung bzw. Erweichung) zur Gesamtoberfläche des Aufnahmeraumes zwischen 1 : 0,35 bis 1 : 0,36 liegt. Ein solches optimales Verhältnis läßt sich durch die gewählte Behälterform erreichen. Demgegenüber ist beispielsweise bei einem kugelförmigen Behälter nur ein Verhältnis von 1 : 0,227 erreichbar, was, wie sich gezeigt hat, nicht ausreicht, um eine effektive Duftabgabe bei vergleichbarem Gesamtvolumen der Partikel zu gewährleisten.

Ferner ist der Behälter bevorzugt so gestaltet, dass die Schichtdicke der Partikel im annähernd vollständig mit Partikeln befüllten Aufnahmeraum zwischen 10 bis 12 mm und das Volumen des Aufnahmeraumes vorzugsweise von 10 bis 500 ml, bevorzugt etwa 40 ml beträgt. Die Volumengröße ist abhängig von dem Einsatzzweck des Duftabgabesystems.

Um das Duftabgabesystem auf einfache Weise, beispielsweise in einer Geschirrspülmaschine, anbringen zu können, weist der Behälter außenseitig eine Aufhängeeinrichtung auf, mit welcher das Duftabgabesystem vorzugsweise in den Oberwagen einer Geschirrspülmaschine eingehängt werden kann. Alternativen sind Befestigungsmittel wie Klebeflächen, mit denen die Vorrichtung an Wänden von Räumen festgelegt werden kann.

Zusätzlich zu den Öffnungen zur Emittierung der Duftstoffe weist der Behälter bevorzugt im Bereich der Rückwand eine Mehrzahl von schlitzförmigen Öffnungen auf, durch welche beim Spülvorgang innerhalb einer Geschirrspülmaschine beispielsweise eine gewisse Menge Feuchtigkeit in das Behälterinnere und aus diesem heraus gelangen kann, was zu einer verbesserten Duftabgabe führen kann.

Unter dem Begriff der "Polymere" werden im Rahmen der vorliegenden Anmeldung Polymerisate, Polyaddukte und Polykondensate zusammengefaßt.

Als Polymerisate werden in dieser Anmeldung solche hochmolekularen Verbindungen bezeichnet, deren Aufbau nach einem Kettenwachstumsmechanismus verläuft. Bevorzugte Polymerisate sind im Rahmen der vorliegenden Anmeldung Polyethylen, Polypropylen, Poly-1-buten, Poly-4-methyl-1-penten, Polyvinylchlorid, Polyvinylidenchforid, Polyacrylnitril und/oder Polystyrol.

Polyaddukte entstehen durch Polyaddition, also Polyreaktionen, bei denen durch sich vielfach wiederholende und voneinander unabhängige Verknüpfungsreaktionen von bis oder polyfunktionellen Edukten (Monomeren) über reaktive Oligomere schließlich Polymere entstehen. Bevorzugte Polyaddukte sind Polyurethane.

Polykondensate entstehen wie die Polyaddukte durch sich vielfach wiederholende und voneinander unabhängige Verknüpfungsreaktionen diskreter Oligomere und Monomere, wobei jedoch im Gegensatz zur Polyaddition gleichzeitig eine Abspaltung niedermolekularer Verbindungen erfolgt. Bevorzugte Polykondensate im Rahmen der vorliegenden Erfindung sind Polyamide, Polycarbonate und Polyester.

Zusammenfassend enthalten die Behälter zur Aufnahme der duftstoffhaltigen Partikel wenigstens anteilsweise Polyethylen, Polypropylen, Polyethylen/Polypropylen-Copolymere, Polyether/Polyamid-Blockcopolymere, Styrol/Butadien-(Block-)Copolymere, Styrol/Isopren-(Block-)Copolymere, Styrol/Ethylen/Butylen-Copolymere, Acrylnitril/Butadien/Styrol-Copolymere, Acrylnitril/Butadien-Copolymere, Polyetherester, Polyisobuten, Polyisopren, Ethylen/Ethylacrylat-Copolymere, Polyamide, Polycarbonat, Polyester, Polyacrylnitril, Polymethyl-methacrylat oder Polyurethane.

Polymere zeichnen sich durch eine besondere Vielseitigkeit auch im Hinblick auf ihre Verarbeitbarkeit aus. Kunststoffe können durch Extrusions- oder Spritzgußverfahren ebenso formgebend verarbeitet werden, wie durch Ziehverfahren. Beim Ziehen (Warmformen) wird eine vorgewärmte Kunststoffplatte oder-folie zwischen die beiden Teile des Werkzeugs, das Positiv und das Negativ, eingeführt und diese dann zusammengedrückt, wodurch das Kunststoffteil seine Form erhält. Ähnlich verläuft die sogenannte Kaltverformung; hier wird die zu verformende Platte bzw. Folie allerdings nicht vorgewärmt. Ist kein Negativ-Werkzeug vorhanden, so spricht man von Tiefziehen. Im Rahmen der vorliegenden Erfindung können die oben genannten Behälter durch, alle dem Fachmann bekannten Verfahren, insbesondere durch Extrusion, Spritzguß, Tiefziehen oder Blasformen hergestellt werden: Die Behälter müssen in jedem Fall den Austritt der Duft- und optionalen anderen Wirkstoffe ermöglichen, wozu sowohl der Einsatz mit Öffnungen versehener Behälter als auch die mindestens anteilsweise Verwendung durchlässiger Behältermaterialien wie permeabler Membranen geeignet ist.

Als "textiles Material" werden im Rahmen der vorliegenden. Anmeldung solche Substanzen bezeichnet, die sich zu textilen Geweben verarbeiten lassen. Zu den bevorzugten textilen Materialien zählen neben den synthetischen Polymeren wie Nylon, Polyester, Polyacryl oder Polyolefine auch die pflanzlichen Materialien wie Baumwolle oder andere cellulosische Materialien.

Die Oberfläche der Feststoffteilchen kann in Abhängigkeit von der Art des gewählten Herstellungsverfahrens und/oder einer gewählten Beschichtung Unebenheiten aufweisen.

Weitere geeignete Behälterformen sind Beutel, Netze, Säckchen, Pouches oder Sachets, welche beispielsweise aus Folien, Vliesen, Geweben oder Gestricken hergestellt sein können und deren Poren- oder Maschenweite ebenso, wie auch bei den vorgenannten Spritzguß-, Blasform- oder Tiefziehkörpern geringer sein muß, als der Durchmesser der Partikel, um ein Herausrieseln zu verhindern.

Die vorgenannten Behälter können nach dem Einfüllen der duftstoffhaltigen Partikel durch Vernähen, Verschweißen oder Verkleben versiegelt werden.

Der Gegenstand der Erfindung ist prinzipiell nicht auf den Einsatz in geschlossenen Räumen beschränkt, sondern eignet sich auch als Duftabgabesystem zur Desodorierung und Beduftung der Raumluft zum Beispiel von Toilettenräumen, wozu das Duftabgabesystem im Bereich der Toilettenschüssel oder deren Umgebung angebracht ist. In der Druckschrift WO 03/042462 A2 ist beispielsweise eine Einrichtung für die Toilettenschüssel mit zwei verschiedenen Kammern beschrieben, von denen eine Kammer eine Reinigungsmittel und eine Kammer ein Duftmittel enthält. Jedoch muss die Kammer für das Reinigungsmittel im Spülstrom des Wassers und die Kammer für das Duftmittel außerhalb desselben angeordnet sein. Durch eine erfindungsgemäße Ausbildung der Duftkammer von Einrichtungen für die Toilettenschüssel ist die Lage der Duftkammer ortsunabhängig, d.h. sie kann außerhalb oder auch im Spülstrom angeordnet sei.

Es wurde gefunden, dass die weitere Aufgabe durch Duftabgabesysteme gelöst werden, welche bestimmte polymere Trägermaterialien aufweisen. Gegenstand der vorliegenden Anmeldung ist daher auch ein Duftabgabesystem, umfassend ein Behältnis sowie Partikel zur Desodorierung und Beduftung von Räumen, welche mindestens ein polymeres Trägermaterial mit einem Schmelz- oder Erweichungspunkt zwischen 30 und 150°C sowie mindestens einen Duftstoff enthalten. Bei diesem Duftabgabesystem werden Partikel verwandt, deren polymeres Trägermaterial einen Schmelz- oder Erweichungspunkt zwischen 30° und 150°C und ganz besonders bevorzugt zwischen 75° und 80°C aufweist. Ein solches Duftabgabesystem verfügt über ein optimiertes Duftfreisetzungsprofil, welches auf einer Veränderung der Verhältnisse von Oberfläche zu Innenvolumen der in dem Duftabgabesystem enthaltenen Partikel beruht. Diese Partikel liegen bei einem fabrikneuen, unverbrauchten System als singuläre Einzelpartikel mit großer Partikeloberfläche vor. Die Zusammensetzung der Partikel ist jedoch so gewählt, dass bei einer thermischen Belastung dieser Partikel das polymere Trägermaterial dieser Partikel oberflächlich erweicht oder schmilzt und einzelne Partikel miteinander unter Verringerung der Gesamtoberfläche verkleben. Die Höhe des Schmelz- oder Erweichungspunktes der polymeren Trägermaterialien wird durch das Einsatzgebiet bestimmt. So treten während des maschinellen Geschirrspülens, insbesondere im Klarspülgang, beispielsweise Höchsttemperaturen zwischen 65° und 75°C auf. Der Behälter dieses Duftabgabesystems besteht vorzugsweise aus einem wasserunlöslichen organischen oder anorganischen Material, wie Kunststoff, Keramik, Glas, Metall oder Textilien.

Solche Materialien zeichnen sich durch eine besondere Vielseitigkeit auch im Hinblick auf ihre Verarbeitbarkeit auf. So können sie durch Extrusions- oder Spritzgussverfahren ebenso formgebend verarbeitet werden wie durch Ziehverfahren. Insbesondere ist eine Herstellung durch Extrusion, Spritzgusstiefziehen oder Blasformen-möglich. Als geometrische Formen für den Behälter können Zylinder, Kugeln, Halbkugeln oder "gestreckte Kugeln" in Form ellipsoider Kapseln, ebenso wie reguläre Polyeder, beispielsweise Tetraeder, Hexaeder, Oktaeder, Dodekaeder, lkosaeder vorgesehen sein. Diese Behälter werden nach der Befüllung mit den Partikeln zur Bildung des Duftabgabesystems verschlossen, um ein Herausrieseln der Partikel auszuschließen. Von Nachteil bei diesem Duftabgabesystem ist jedoch die bisher gewählte Behälterform, da bei diesen Behältern noch kein optimales Verhältnis zwischen dem Gesamtvolumen der Partikel und der Oberfläche des Partikelkonglomerates erreicht werden konnte.

Die verbesserte Duftfreisetzung erfindungsgemäßer Duftabgabesysteme beruht dabei auf einer Veränderung der Verhältnisses von Oberfläche zu Innenvolumen der in dem Duftabgabesystem enthaltenen Partikel. Diese Partikel liegen bei einem fabrikneuen, unverbrauchten System als singuläre Partikel mit großer Partikeloberfläche vor. Die Zusammensetzung der Partikel ist jedoch so gewählt, daß bei einer thermischen Belastung dieser Partikel das polymere Trägermaterial dieser Partikel oberflächlich erweicht oder schmilzt und einzelne Partikel miteinander unter Verringerung der Gesamtoberfläche verkleben. Die Höhe des Schmelz- oder Erweichungspunktes der polymeren Trägermaterialien wird demnach durch das Einsatzgebiet erfindungsgemäßer Mittel bestimmt. So treten während des maschinellen Geschirrspülens, insbesondere im Klarspülgang, beispielsweise Höchsttemperaturen zwischen 65 und 75°C auf. Dienen erfindungsgemäße Mittel der Raumbeduftung in Gebäuden oder Fahrzeugen, beispielsweise als Aufsatz für Heizungen, so liegen die dort erreichten Höchsttemperaturen in der Regel im Bereich von 70 bis 90°C. In jedem Fall sollten die Schmelz- oder Erweichungspunkte der Partikel oberhalb der bei deren Transport und Lagerung üblichen Umgebungstemperatur und unterhalb der Zersetzungstemperaturen der enthaltenen Duftstoffe liegen.

Als polymeres Trägermaterial für die duftstoffhaltigen Partikel eignen sich generell alle Polymere oder Polymergemische, welche die oben genannten Kriterien bezüglich der Schmelz- oder Erweichungstemperatur erfüllen. Im Rahmen der vorliegenden Anmeldung bevorzugte Duftabgabesysteme sind dadurch gekennzeichnet, daß das polymere Trägermaterial mindestens eine Substanz aus der Gruppe umfassend EthylenNinylacetat- Copolymere, Polyethylen niederer oder hoher Dichte (LDPE, HDPE) oder Gemische derselben, Polypropylen, Polyethylen/Polypropylen-Copolymere, Polyether/Polyamid-Blockcopolymere, Styrol/Butadien-(Block-)Copolymere, Styrol/Isopren-(Block-)Copolymere, Styrol/Ethylen/Butylen-Copolymere, Acrylnitril/Butadien/Styrol-Copolymere, Acrylnitril/Butadien-Copolymere, Polyetherester, Polyisobuten, Polyisopren, Ethylen/Ethylacrylat-Copolymere, Polyamide, Polycarbonat, Polyester, Polyacrylnitril, Polymethyl-methacrylat, Polyurethane, Polyvinylalkohole enthält.

Polyethylen (PE) ist eine Sammelbezeichnung für zu den Polyolefinen gehörende Polymere mit Gruppierungen des Typs

CH₂-CH₂

als charakteristische Grundeinheit der Polymerkette. Polyethylene werden in der Regel durch Polymerisation von Ethylen nach zwei grundsätzlich unterschiedlichen Methoden, dem Hochdruck- und dem Niederdruck-Verfahren hergestellt. Die resultierenden Produkte werden entsprechend häufig als Hochdruck-Polyethylene bzw. Niederdruck-Polyethylene bezeichnet; sie unterscheiden sich hauptsächlich hinsichtlich ihres Verzweigungsgrades und damit verbunden in ihrem Kristallinitätsgrad und ihrer Dichte. Beide Verfahren können als Lösungspolymerisation, Emulsionspolymerisation oder Gasphasenpolymerisation durchgeführt werden.

Beim Hochdruck-Verfahren fallen verzweigte Polyethylene mit niedriger Dichte (ca. 0,915-0,935 g/cm³) und Kristallinitätsgraden von ca. 40-50% an, die man als LDPE-Typen (low density polyethylene) bezeichnet. Produkte mit höherer Molmasse und dadurch bedingter verbesserter Festigkeit und Streckbarkeit tragen die Kurzbezeichnung HMW-LDPE (HMW=high molecular weight). Durch Copolymerisation des Ethylens mit längerkettigen Olefinen, insbesondere mit Buten und Octen, kann der ausgeprägte Verzweigungsgrad der im Hochdruck-Verfahren hergestellten Polyethylene reduziert werden; die Copolymere haben das Kurzzeichen LLD-PE (linear low density polyethylene).

Die Makromoleküle der Polyethylene aus Niederdruck-Verfahren sind weitgehend linear und unverzweigt. Diese Polyethylene, Kurzzeichen HDPE (von E high density polyethylene) haben Kristallinitätsgrade von 60-80% und eine Dichte von ca. 0,94-0,965 g/cm3. Sie werden als Produkte mit hoher bzw. ultrahoher Molmasse (ca. 200 000-5 000 000 g/mol bzw. 3 000 000-6 000 000 g/mol) unter der Kurzbezeichnung HD-HMW-PE bzw. UHMW-HD-PE angeboten. Auch Produkte mit mittlerer Dichte (MDPE) aus Mischungen von Polyethylenen niedriger und hoher Dichte sind kommerziell erhältlich. Lineare Polyethylene mit Dichten <0,918 g/cm3 (VLD-PE, von E very low density polyethylene) gewinnen nur langsam Marktbedeutung.

Polyethylene haben eine sehr geringe Wasserdampfdurchlässigkeit, die Diffusion von Gasen sowie von Aromastoffen und etherischen Substanzen durch Polyethylene ist relativ hoch. Die mechanischen Eigenschaften sind stark abhängig von Molekülgröße und -struktur der Polyethylene. Generell steigen Kristallinitätsgrad und Dichte von Polyethylene mit abnehmendem Verzweigungsgrad und mit Verkürzung der Seitenketten an. Mit der Dichte steigen Schubmodul, Härte, Streckgrenze und Schmelzbereich; es nehmen ab Schockfestigkeit, Transparenz, Quellbarkeit und Löslichkeit. Bei gleicher Dichte nehmen mit steigender Molmasse der Polyethylene Reißfestigkeit, Dehnung, Schockfestigkeit, Schlagzähigkeit und Dauerstandfestigkeit zu. Je nach Arbeitsweise bei der Polymerisation kann man Produkte mit Paraffinwachs-ähnlichen Eigenschaften (MR um 2000) und Produkte mit höchster Zähigkeit (MR über 1 Mio.) erhalten.

Die Verarbeitung der Polyethylen-Typen kann nach allen für Thermoplaste üblichen Methoden erfolgen.

Polypropylen (PP) ist die Bezeichnung für thermoplastische Polymere des Propylens mit der allg. Formel:

-(CH₂-CH[CH3])ₙ-

Basis für die Polypropylen-Herstellung war die Entwicklung des Verfahrens zur stereospezifischen Polymerisation von Propylen in der Gasphase oder in Suspension durch Natta. Diese wird mit Ziegler-Natta-Katalysatoren, in zunehmendem Maße aber auch durch Metallocen-Katalysatoren initiiert und führt entweder zu hochkristallinen isotaktischen oder zu weniger kristallinen syndiotaktischen bzw. zu amorphen ataktischen Polypropylenen.

Polypropylen zeichnet sich durch hohe Härte, Rückstellfähigkeit, Steifheit und Wärmebeständigkeit aus. Kurzfristiges Erwärmen von Gegenständen aus Polypropylen ist sogar bis 140 °C möglich. Bei Temperaturen unter 0 °C tritt eine gewisse Versprödung der Polypropylene ein, die jedoch durch Copolymerisation des Propylens mit Ethylen (EPM, EPDM) zu wesentlich tieferen Temperaturbereichen verschoben werden kann. Allgemein läßt sich die Schlagzähigkeit von Polypropylen durch Modifikation mit Elastomeren verbessern. Die Chemikalienbeständigkeit ist wie bei allen Polyolefinen gut. Eine Verbesserung der mechanische Eigenschaften der Polypropylene erreicht man durch Verstärkung mit Talkum, Kreide, Holzmehl oder Glasfasern. Polypropylene sind in noch stärkerem Maße als PE oxidations- und lichtempfindlich, weshalb der Zusatz von Stabilisatoren (Antioxidantien, Lichtschutzmittel, UV-Absorber) erforderlich ist.

Polyether ist eine auf dem Gebiet der Makromolekularen Chemie übergreifende Bezeichnung für Polymere, deren organische Wiederholungseinheiten durch Ether-Funktionalitäten (C-O-C) zusammengehalten werden. Nach dieser Definition gehört eine Vielzahl strukturell sehr unterschiedlicher Polymerer zu den Polyethern, z. B. die Polyalkylenglykole (Polyethylenglykole, Polypropylenglykole und Polyepichlorhydrine) als Polymere von 1,2-Epoxiden, Epoxidharze, Polytetrahydrofurane (Polytetramethylenglykole), Polyoxetane, Polyphenylenether (s. Polyarylether) oder Polyetheretherketone (s. Polyetherketone). Nicht zu den Polyethern werden Polymere mit seitenständigen Ether-Gruppen gerechnet, wie u. a. die Celluloseether, Stärkeether und Vinylether-Polymere.

Zur Gruppe der Polyether zählen weiterhin auch funktionalisierte Polyether, d. h. Verbindungen mit einem Polyether-Gerüst, die an ihren Hauptketten seitlich angeheftet noch andere funktionelle Gruppen tragen wie z. B. Carboxy-, Epoxy-, Allyl- oder Amino-Gruppen usw. Vielseitig verwendbar sind Block-Copolymere von Polyethern und Polyamiden (sog. Polyetheramide oder Polyether-Blockamide, PEBA).

Als Polyamide (PA) werden Polymere bezeichnet, deren Grundbausteine durch Amid-Bindungen (-NH-CO-) zusammengehalten werden. Natürlich vorkommende Polyamide sind Peptide, Polypeptide und Proteine (Beisp.: Eiweiß, Wolle, Seide). Die synthetischen Polyamide sind bis auf wenige Ausnahmen thermoplastische, kettenförmige Polymere, von denen einige große technische Bedeutung als Synthesefasern und Werkstoffe erlangt haben. Nach dem chemischen Aufbau lassen sich die sogenannte Homo-Polyamide in zwei Gruppen einteilen, die Aminocarbonsäure-Typen (AS) und die Diamin-Dicarbonsäure-Typen (AA-SS; dabei bezeichnen A Amino-Gruppen und S Carboxy-Gruppen). Erstere werden aus nur einem einzigen Monomeren durch z. B. Polykondensation einer ω-Aminocarbonsäure (1) (Polyaminosäuren) oder durch ringöffnende Polymerisation cyclischer Amide (Lactame) (2) hergestellt.

Neben den Homopolyamiden haben auch einige Co-Polyamide Bedeutung erlangt. Üblich ist bei diesen eine qualitative und quantitative Angabe der Zusammensetzung z. B. PA 66/6 (80:20) für aus 1,6-Hexandiamin, Adipinsäure und ε-Caprolactam im Molverhältnis 80:80:20 hergestellte Polyamide.
Wegen ihrer besonderen Eigenschaften werden Polyamide, die ausschließlich aromatische Reste enthalten (z. B. solche aus p-Phenylendiamin und Terephthalsäure), unter der Gattungsbez. Aramide oder Polyaramide zusammengefaßt (Beisp.: Nomex®).

Die am häufigsten verwendeten Polyamid-Typen (v. a. PA 6 und PA 66) bestehen aus unverzweigten Ketten mit mittleren Molmassen von 15 000 bis 50 000 g/mol. Sie sind im festen Zustand teilkristallin und haben Kristallisationsgrade von 30-60%.

Eine Ausnahme bilden Polyamide aus Bausteinen mit Seitenketten oder Co-Polyamide aus stark unterschiedlichen Komponenten, die weitgehend amorph sind. Im Gegensatz zu den im allgemeinen milchig-opaken, teilkristallinen Polyamiden sind diese fast glasklar. Die Erweichungstemperatur der gebräuchlichsten Homo-Polyamide liegen zwischen 200 und 260 °C (PA-6: 215-220 °C, PA 66: 255-260 °C).

Polyester ist die Sammelbezeichnung für Polymere, deren Grundbausteine durch Ester-Bindungen (-CO-O-) zusammengehalten werden. Nach ihrem chemischen Aufbau lassen sich die sogenannte Homopolyester in zwei Gruppen einteilen, die Hydroxycarbonsäüre-Typen (AB-Polyester) und die Dihydroxy-Dicarbonsäure-Typen (AA-BB-Polyester). Erstere werden aus nur einem einzigen Monomer durch z. B. Polykondensation einer ω-Hydroxycarbonsäure, 1 oder durch Ringöffnungspolymerisation cyclischer Ester (Lactone) 2 hergestellt.

Verzweigte und vernetzte Polyester werden bei der Polykondensation von drei- oder mehrwertigen Alkoholen mit polyfunktionellen Carbonsäuren erhalten. Zu den Polyestern werden allgemein auch die Polycarbonate (Polyester der Kohlensäure) gerechnet. AB-Typ-Polyester (I) sind u. a. Polyglykolsäuren, Polymilchsäuren, Polyhydroxybuttersäure [Poly(3-hydroxybuttersäure), Poly(ε-caprolacton)e und Polyhydroxybenzoesäuren.

Rein aliphatische AA-BB-Typ-Polyester (II) sind Polykondensate aus aliphatischen Diolen und Dicarbonsäuren, die u. a. als Produkte mit endständigen HydroxyGruppen (als Polydiole) für die Herstellung von Polyesterpolyurethanen eingesetzt werden [z. B. Polytetramethylenadipat]. Mengenmäßig die größte technische Bedeutung haben AA-BB-Typ-Polyester aus aliphatischen Diolen und aromatischen Dicarbonsäuren, insbesondere die Polyalkylenterephthalate, mit Polyethylenterephthalat (PET), Polybutylenterephthalat (PBT) und Poly(1,4-cyclohexandimethylenterephthalat)e (PCDT) als wichtigste Vertreter. Diese Typen von Polyestern können durch Mitverwenden anderer aromatischer Dicarbonsäuren (z. B. Isophthalsäure) bzw. durch Einsatz von Diol-Gemischen bei der Polykondensation in ihren Eigenschaften breit variiert und unterschiedlichen Anwendungsgebieten angepaßt werden.

Rein aromatische Polyester sind die Polyarylate, zu denen u. a. die Poly(4-hydroxybenzoesäure) gehören. Zusätzlich zu den bisher genannten gesättigten Polyestern lassen sich auch ungesättigte Polyester aus ungesättigten Dicarbonsäuren herstellen, die als Polyesterharze, insbesondere als ungesättigte Polyesterharze (UP-Harze), technische Bedeutung erlangt haben.

Polyester sind in der Regel Thermoplaste. Produkte auf der Basis von aromatischen Dicarbonsäuren besitzen ausgesprochenen Werkstoffcharakter. Die rein aromatischen Polyarylate zeichnen sich durch hohe Thermostabilität aus.

Als Polyurethane (PUR) werden Polymere bezeichnet, in deren Makromolekülen die Wiederholungseinheiten durch Urethan-Gruppierungen -NH-CO-O-verknüpft sind. Polyurethane werden im allgemeinen durch Polyaddition aus zwei- oder höherwertigen Alkoholen und Isocyanaten erhalten.

Je nach Wahl und stöchiometrischem Verhältnis der Ausgangsstoffe entstehen so Polyurethane mit sehr unterschiedlichen mechanischen Eigenschaften, die als Bestandteile von Klebstoffen und Lacken (Polyurethan-Harze), als lonomere, als thermoplastisches Material für Lagerteile, Rollen, Reifen, Walzen verwendet werden und als mehr oder weniger harte Elastomere in Faserform (Elastofasern, Kurzz. PUE für diese Elastan- oder Spandex-Fasern) oder als Polyether- bzw. Polyesterurethan-Kautschuk (EU bzw. AU)

Polyurethan-Schäume entstehen bei der Polyaddition, wenn Wasser und/oder Carbonsäuren zugegen sind, denn diese reagieren mit den Isocyanaten unter Abspaltung von auftreibend und Schaum-bildend wirkendem Kohlendioxid. Mit Polyalkylenglykolethern als Diolen und Wasser als Reaktionskomponente gelangt man zu Polyurethan-Weichschäumen, mit Polyolen und Treibgasen aus FCKW (bes. R 11) erhält man Polyurethan-Hartschaumstoffe und Struktur- oder Integralschaumstoffe. Zusätzlich benötigte Hilfsstoffe sind hier z. B. Katalysatoren, Emulgatoren, Schaumstabilisatoren (bes. Polysiloxan-Polyether-Copolymere), Pigmente, Alterungs- und Flammschutzmittel. Zur Herst. von auch kompliziert geformten Gegenständen aus Polyurethan-Schaum hat man in den 70er Jahren die sogenannte RIM-Technik entwickelt (reaction injection molding = Reaktionsspritzguß). Das RIM-Verf. beruht auf raschem Dosieren und Mischen der Komponenten, Injektion des reaktiven Gemisches in die Form und schnellem Aushärten; die Cycluszeit beträgt nur wenige Minuten. Mittels RIM-Technik werden u. a. Autokarosserieteile, Schuhsohlen, Fensterprofile und Fernsehgehäuse erzeugt.

Polyvinylalkohole (PVAL, gelegentlich auch PVOH) ist dabei die Bezeichnung für Polymere der allgemeinen Struktur 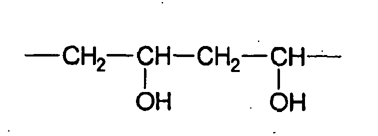 die in geringen Anteilen (ca. 2%) auch Struktureinheiten des Typs 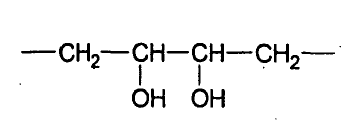 enthalten.

Handelsübliche Polyvinylalkohole werden als weiß-gelbliche Pulver oder Granulate mit Polymerisationsgraden im Bereich von ca. 100 bis 2500 (Molmassen von ca. 4000 bis 100.000 g/mol) angeboten. Charakterisiert werden die Polyvinylalkohole von Seiten der Hersteller durch Angabe des Polymerisationsgrades des Ausgangspolymeren, des Hydrolysegrades, der Verseifungszahl bzw. der Lösungsviskosität.

Polyvinylalkohole sind abhängig vom Hydrolysegrad löslich in Wasser und wenigen stark polaren organischen Lösungsmitteln (Formamid, Dimethylformamid, Dimethylsulfoxid); von (chlorierten) Kohlenwasserstoffen, Estern, Fetten und Ölen werden sie nicht angegriffen. Polyvinylalkohole werden als toxikologisch unbedenklich eingestuft und sind biologisch zumindest teilweise abbaubar. Die Wasserlöslichkeit kann man durch Nachbehandlung mit Aldehyden (Acetalisierung), durch Komplexierung mit Ni- oder Cu-Salzen oder durch Behandlung mit Dichromaten, Borsäure oder Borax verringern. Die Beschichtungen aus Polyvinylalkohol sind weitgehend undurchdringlich für Gase wie Sauerstoff, Stickstoff, Helium, Wasserstoff, Kohlendioxid, lassen jedoch Wasserdampf hindurchtreten.

Vorzugsweise werden als Materialien für die Behälter Polyvinylalkohole eines bestimmten Molekulargewichtsbereichs eingesetzt, wobei erfindungsgemäß bevorzugt ist, daß der wasserlösliche oder wasserdispergierbare Behälter einen Polyvinylalkohol umfaßt, dessen Molekulargewicht im Bereich von 10.000 bis 100.000 gmol⁻¹, vorzugsweise von 11.000 bis 90.000 gmol⁻¹, besonders bevorzugt von 12.000 bis 80.000 gmol⁻¹ und insbesondere von 13.000 bis 70.000 gmol⁻¹ liegt.

In einer besonders bevorzugten Ausführungsform der vorliegenden Erfindung besteht das polymere Trägermaterial der Partikel wenigstens anteilsweise aus EthylenNinylacetat-Copolymer. Ein weiterer bevorzugter Gegenstand der vorliegenden Anmeldung ist daher ein Duftabgabesystem, dadurch gekennzeichnet, daß das polymere Trägermaterial mindestens 10 Gew.-%, vorzugsweise mindestens 30 Gew.-%, besonders bevorzugt mindestens 70 Gew.-% EthylenNinylacetat-Copolymer enthält, vorzugsweise vollständig aus EthylenNinylacetat-Copolymer hergestellt ist.

EthylenNinylacetat-Copolymere ist die Bezeichnung für Copoylmere aus Ethylen und Vinylacetat. Die Herstellung dieses Polymers erfolgt grundsätzlich in einem der Herstellung von Polyethylen mit niedriger Dichte (LDPE; low density polyethylene) vergleichbaren Verfahren. Mit einem zunehmenden Anteil an Vinylacetat wird die Kristallinität des Polyethylens unterbrochen und auf diese Weise die Schmelz- und Erweichungspunkte bzw. die Härte der resultierenden Produkte herabgesetzt. Das Vinylacetat macht das Copolymer zudem polarer und verbessert damit dessen Adhäsion an polare Substrate.

Die vorstehend beschriebenen EthylenNinylacetat-Copolymere sind kommerziell breit verfügbar, beispielsweise unter dem Warenzeichen Elvax^{®} (Dupont). Im Rahmen der vorliegenden Erfindung besonders geeignete Polyvinylalkohole sind beispielsweise Elvax^{®} 265, Elvax^{®} 240, Elvax^{®} 205 W, Elvax^{®} 200 W sowie Elvax^{®} 360.

Einige besonders geeignete Copolymere und deren physikalische Eigenschaften sind der nachstehenden Tabelle zu entnehmen:

| Produktname | Gew.-% Vinylacetat (bezogen auf das Gesamtgewicht) | Schmelzpunkt |
|---|---|---|
| Elvax^{®} 40W | 40 | 47°C |
| Elvax^{®} 150 | 33 | 63°C |
| Elvax^{®} 265 | 28 | 75°C |
| Elvax^{®} 240 | 28 | 74°C |
| Elvax^{®} 205 W | 28 | 72°C |
| Elvax^{®} 200 W | 28 | 71°C |
| Elvax^{®} 360 | 25 | 78°C |
| Elvax^{®} 460 | 18 | 88°C |
| Elvax^{®} 660 | 12 | 96°C |
| Elvax^{®} 760 | 9 | 100°C |

Im Rahmen der vorliegenden Erfindung, insbesondere im Bereich der Beduftung der Innenräume von maschinellen Geschirrspülmaschinen sind Duftabgabesysteme besonders bevorzugt, in welchen als polymeres Trägermaterial EthylenNinylacetat-Copolymer eingesetzt wird und dieses Copolymer 5 bis 50 Gew.-% Vinylacetat, vorzugsweise 10 bis 40 Gew.-% Vinylacetat und insbesondere 20 bis 30 Gew.-% Vinylacetat, jeweils bezogen auf das Gesamtgewicht des Copolymers, enthält.

Erfindungsgemäße Duftabgabesysteme enthalten die polymeren Trägermaterialien in Form von Partikeln. Die Raumform dieser Partikel wird lediglich durch die technischen Möglichkeiten bei deren Herstellung beschränkt. Als Raumform kommen damit alle sinnvoll handhabbaren Ausgestattungen in Betracht, beispielsweise also Würfel, Quader und entsprechende Raumelemente mit ebenen Seitenflächen sowie insbesondere zylinderförmige Ausgestaltungen mit kreisförmigem oder ovalem Querschnitt. Diese letzte Ausgestaltung umfaßt dabei tablettenförmige Teilchen bis hin zu kompakten Zylinderstücken mit einem Verhältnis von Höhe zu Durchmesser oberhalb 1. Weitere mögliche Raumformen sind Kugeln, Halbkugeln oder "gestreckte Kugeln" in Form ellipsoider Kapseln ebenso wie reguläre Polyeder, beispielsweise Tetraeder, Hexaeder, Oktaeder, Dodekaeder, Ikosaeder. Denkbar sind weiterhin sternförmige Ausbildungen mit drei, vier, fünf, sechs oder mehr Spitzen oder völlig unregelmäßige Körper, die beispielsweise motivisch ausgestaltet sein können. Als Motive eignen sich in Abhängigkeit von dem Einsatzgebiet der erfindungsgemäßen Mittel zum Beispiel Tierfiguren, wie Hunde, Pferde oder Vögel, florale Motive oder die Darstellung von Früchten. Die motivische Ausgestaltung kann sich aber auch auf unbelebte Gegenstände wie Fahrzeuge, Werkzeuge, Haushaltsgegenstände oder Bekleidung beziehen. Die Oberfläche der Feststoffteilchen kann in Abhängigkeit von der Art des gewählten Herstellungsverfahrens und/oder einer gewählten Beschichtung Unebenheiten aufweisen. Aufgrund der zahlreichen Ausgestaltungsmöglichkeiten für die Partikel zeichnen sich die erfindungsgemäßen Mittel nicht nur durch Vorteile bei ihrer Herstellung aus. Aufgrund der vielfältigen Ausgestaltungsformen sind die duftstoffhaltigen Partikel zudem für den Verbraucher optisch deutlich wahrnehmbar und ermöglichen durch die gezielte räumliche Gestaltung dieser Partikel ein für die Produktakzeptanz - besonders vorteilhafte Visualisierung der in den erfindungsgemäßen Mittel enthaltenen Duftstoffe, bzw. weiterer optional in diesen Mitteln enthaltenen Aktivsubstanzen. So kann durch die optisch wahrnehmbare Mehrphasigkeit dieser Mittel beispielsweise die unterschiedliche Wirkweise einzelnerAktivsubstanzen (z.B. Reinigungs- und Zusatzfunktionen wie Glasschutz, Silberschutz etc.) verdeutlicht werden.

Als Partikel werden im Rahmen der vorliegenden Anmeldung Teilchen zusammengefaßt, welche eine bei Raumtemperatur feste, das heißt formstabile, nicht fließfähige Konsistenz aufweisen. Bevorzugte Partikel weisen einen mittleren Durchmesser von 0,5 bis 20 mm, vorzugsweise von 1 bis 10 mm und insbesondere von 3 bis 6 mm auf.

Die Konfektionierung der polymeren Trägermaterialien zu den zuvor beschriebenen Partikeln kann auf alle dem Fachmann zur Verarbeitung dieser Substanzen bekannten Verfahren erfolgen. Bevorzugt werden im Rahmen der vorliegenden Erfindung die Extrusion das Spritzgießen und das Versprühen zu Polymergranulaten bevorzugt.

Erfindungsgemäße Duftabgabesysteme umfassen neben einem Behältnis weiterhin duftstoffhaltige Partikel auf Basis polymerer Trägermaterialien, wobei der Gewichtsanteil des/der Duftstoffe(s), bezogen auf das Gesamtgewicht der Partikel, vorzugsweise 1 bis 70 Gew.-%, vorzugsweise 10 bis 60 Gew.-%, besonders bevorzugt 20 bis 50 Gew.-%, insbesondere 30 bis 40 Gew.-%, beträgt.

Als Parfümöle bzw. Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, ∝-tsomethytionon und Methyl-cedrylketon, zu den Alkoholen Anethol, Citronellol, Eugenol, Geraniol, Linalool, Phenyl ethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Die allgemeine Beschreibung der einsetzbaren Parfüme (siehe oben) stellt dabei allgemein die unterschiedlichen Substanzklassen von Riechstoffen dar. Um wahrnehmbar zu sein, muß ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Auf Grund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Durch eine geeignete Auswahl der genannten Duftstoffe bzw. Parfümöle kann auf diese Weise für erfindungsgemäße Mittel sowohl der Produktgeruch unmittelbar beim Öffnen des fabrikneuen Mittels als auch der Gebrauchsduft, beispielsweise beim Einsatz in einer Geschirrspülmaschine, beeinflußt werden. Diese Dufteindrücke können selbstverständlich gleich sein, können sich aber auch unterscheiden. Für den letzteren Geruchseindruck ist die Verwendung haftfesterer Riechstoffe vorteilhaft, während zur Produktbeduftung auch leichterflüchtige Riechstoffe einsetzbar sind. Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethytether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester. Zu den leichter flüchtigen Riechstoffen zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Bevorzugt werden die Kunststoffpartikel bei einer Temperatur von 15 bis 30°C, vorzugsweise von 20 bis 25°C, mit dem ausgewählten Duftstoff beladen. Hierzu werden die Partikel mit der entsprechenden Menge des Duftstoffs versetzt und durchmischt. In jedem Fall sollte die Temperatur aber unterhalb der Schmelz- oder Zersetzungstemperatur des Kunststoffs und auch unterhalb des Flammpunkts des Parfumöls liegen. Der Duftstoff wird vorrangig durch Adhäsions-, Diffusions- und/oder Kapillarkräfte vom polymeren Trägermaterial oder von im Partikel enthaltenen weiteren Parfümträgermaterialien aufgenommen, wobei diese im Laufe dieses Vorgangs geringfügig quellen können.

Wie zuvor erwähnt, können erfindungsgemäße Mittel außer den zur Beduftung und Desodorierung notwendigen Bestandteilen weitere Aktivsubstanzen enthalten. Von den Mitteln, welche ausschließlich der Beduftung dienen, lassen sich demnach weitere Produktgruppen unterscheiden, welche zusätzlich zu den vorgenannten erfindungsgemäßen Bestandteilen weitere bevorzugte Substanzen enthalten.

Eine erste dieser optional einsetzbaren bevorzugten Substanzen sind die Farbstoffe. Hierzu eignen sich generell sämtliche Farbstoffe, die dem Fachmann als geeignet zum Einfärben von Kunststoffen bzw. als löslich in Parfumölen bekannt sind. Es ist bevorzugt, den Farbstoff entsprechend des verwendeten Duftstoffs auszuwählen; beispielsweise weisen Partikel mit Zitronenduft vorzugsweise eine gelbe Farbe auf, während für Partikel mit Apfel- oder Kräuterduft eine grüne Farbe bevorzugt wird. Bevorzugte Farbstoffe besitzen eine hohe Lagerstabilität und Unempfindlichkeit gegenüber den übrigen Inhaltsstoffen der Mittel und gegen Licht. Werden die erfindungsgemäßen Mittel im Zusammenhang mit der Textil- oder Geschirreinigung eingesetzt, sollten die eingesetzten Farbstoffe keine ausgeprägte Substantivität gegenüber Textilfaser, Glas, Kunststoffgeschirr oder Keramik aufweisen, um diese nicht anzufärben.

Geeignete Farbstoffe und Farbstoffgemische sind unter verschiedenen Handelsnamen kommerziell erhältlich und werden unter anderem von den Firmen BASF AG, Ludwigshafen, Bayer AG, Leverkusen, Clariant GmbH, DyStar Textilfarben GmbH & Co. Deutschland KG, Les Colorants Wackherr SA und Ciba Specialty Chemicals angeboten. Zu den geeigneten fettlöslichen Farbstoffen und Farbstoffgemischen zählen beispielsweise Solvent Blue 35, Solvent Green 7, Solvent Orange 1 (Orange au Gras-W-2201), Sandoplast Blau 2B, Fettgelb 3G, Iragon® Red SRE 122, Iragon® Green SGR 3, Solvent Yellow 33 und Solvent Yellow 16, es können aber auch andere Farbstoffe enthalten sein.

In einer bevorzugten Ausführungsform besitzt der Farbstoff neben seiner ästhetischen Wirkung zusätzlich eine Indikatorfunktion. Hierdurch wird dem Konsumenten der aktuelle Verbrauchszustand des Deodorants angezeigt, so daß er neben dem fehlenden Dufteindruck, der beispielsweise auch auf einem Gewöhnungseffekt seitens des Benutzers beruhen kann, ein weiteres zuverlässiges Anzeichen erhält, wann das Deodorant durch ein neues zu ersetzen ist.

Die Indikatorwirkung kann auf verschiedenen Wegen erzielt werden: Einerseits kann ein Farbstoff verwendet werden, der im Laufe der Anwendungsdauer aus den Partikeln entweicht. Dies kann zum Beispiel durch die im Geschirrspülmittel enthaltenen Inhaltsstoffe bewirkt werden. Hierzu muß ein Farbstoff eingesetzt werden, der gut an den Partikeln haftet bzw. nur langsam aus ihnen herausdiffundiert, um zu gewährleisten, daß die Entfärbung nicht zu früh, nämlich wenn der Duftstoff noch nicht verbraucht ist, beendet ist. Andererseits kann aber auch durch eine chemische Reaktion oder thermische Zersetzung ein Farbumschlag hervorgerufen werden.

Weitere bevorzugte Bestandteile erfindungsgemäßer Mittel sind Substanzen wie antimikrobielle Wirkstoffe, Germizide, Fungizide, Antioxidantien oder Korrosionsinhibitoren, mit deren Hilfe sich Zusatznutzen, wie beispielsweise die Desinfektion oder der Korrosionsschutz realisieren lassen.

Zur Bekämpfung von Mikroorganismen können die erfindungsgemäßen Mittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw.. Wichtige Stoffe aus diesen Gruppen sind beispielsweise Benzalkoniumchloride, Alkylarlylsulfonate, Halogenphenole und Phenolmercuriacetat.

Um unerwünschte, durch Sauerstoffeinwirkung und andere oxidative Prozesse verursachte Veränderungen an den erfindungsgemäßen Mitteln oder den beispielsweise behandelten Textilien zu verhindern, können die Mittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite und Phosphonate.
Werden die erfindungsgemäßen Mittel in Geschirrspülmaschinen zum Einsatz gebracht, so können diese Mittel zum Schutze des Spülgutes oder der Maschine Korrosionsinhibitoren enthalten, wobei besonders Silberschutzmittel im Bereich des maschinellen Geschirrspülens eine besondere Bedeutung haben. Einsetzbar sind die bekannten Substanzen des Standes der Technik Allgemein können vor allem Silberschutzmittel ausgewählt aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zu verwenden sind Benzotriazol und/oder Alkylaminotriazol. Man findet in Reinigerformulierungen darüber hinaus häufig aktivchlorhaltige Mittel, die das Korrodieren der Silberoberfläche deutlich vermindern können. In chlorfreien Reinigern werden besonders sauerstoff- und stickstoffhaltige organische redoxaktive Verbindungen, wie zwei- und dreiwertige Phenole, z. B. Hydrochinon, Brenzkatechin, Hydroxyhydrochinon, Gallussäure, Phloroglucin, Pyrogallol bzw. Derivate dieser Verbindungsklassen, eingesetzt. Auch salz- und komplexartige anorganische Verbindungen, wie Salze der Metalle Mn, Ti, Zr, Hf, V, Co und Ce finden häufig Verwendung. Bevorzugt sind hierbei die Übergangsmetallsalze, die ausgewählt sind aus der Gruppe der Mangan und/oder Cobaltsalze und/oder - komplexe, besonders bevorzugt der Cobalt(ammin)-Komplexe, derCobalt(acetat)-Komplexe, der Cobalt-(Carbonyl)-Komplexe, der Chloride des Cobalts oder Mangans und des Mangansulfats. Ebenfalls können Zinkverbindungen zur Verhinderung der Korrosion am Spülgut eingesetzt werden.

Anstelle von oder zusätzlich zu den vorstehend beschriebenen Silberschutzmitteln, beispielsweise den Benzotriazolen, können in den erfindungsgemäßen Mittel redoxaktive Substanzen eingesetzt werden. Diese Substanzen sind vorzugsweise anorganische redoxaktive Substanzen aus der Gruppe der Mangan-, Titan-, Zirkonium-, Hafnium-, Vanadium-, Cobalt- und Cer-Salze und/oder -Komplexe, wobei die Metalle vorzugsweise in einer der Oxidationsstufen II, III, IV, V oder VI vorliegen.

Die verwendeten Metallsalze bzw. Metallkomplexe sollen zumindest teilweise in Wasser löslich sein. Die zur Salzbildung geeigneten Gegenionen umfassen alle üblichen ein-, zwei-, oder dreifach negativ geladenen anorganischen Anionen, z. B. Oxid, Sulfat, Nitrat, Fluorid, aber auch organische Anionen wie z. B. Stearat.

Metallkomplexe im Sinne der Erfindung sind Verbindungen, die aus einem Zentralatom und einem oder mehreren Liganden sowie gegebenenfalls zusätzlich einem oder mehreren der o.g. Anionen bestehen. Das Zentralatom ist eines der o.g. Metalle in einer der o.g. Oxidationsstufen. Die Liganden sind neutrale Moleküle oder Anionen, die ein- oder mehrzähnig sind; der Begriff "Liganden" im Sinne der Erfindung ist z.B. in "Römpp Chemie Lexikon, Georg Thieme Verlag Stuttgart/New York, 9. Auflage, 1990, Seite 2507" näher erläutert. Ergänzen sich in einem Metallkomplex die Ladung des Zentralatoms und die Ladung des/der Liganden nicht auf Null, so sorgt, je nachdem, ob ein kationischer oder ein anionischer Ladungsüberschuß vorliegt, entweder eines oder mehrere der o.g. Anionen oder ein oder mehrere Kationen, z. B. Natrium-, Kalium-, Ammoniumionen, für den Ladungsausgleich. Geeignete Komplexbildner sind z.B. Citrat, Acetylacetonat oder 1-Hydroxyethan-1,1-diphosphonat.

Die in der Chemie geläufige Definition für "Oxidationsstufe" ist z.B. in "Römpp Chemie Lexikon, Georg Thieme Verlag Stuttgart/New York, 9. Auflage, 1991, Seite 3168" wiedergegeben.

Besonders bevorzugte Metallsalze und/oder Metallkomplexe sind ausgewählt aus der Gruppe MnSO₄, Mn(II)-citrat, Mn(II)-stearat, Mn(II)-acetylacetonat, Mn(II)-[1-Hydroxyethan-1,1-diphosphonat], V₂O₅, V₂O₄, VO₂, TiOSO₄, K₂TiF₆, K₂ZrF₆, CoSO₄, Co(NO₃)₂, Ce(NO₃)₃ sowie deren Gemische, so daß bevorzugte erfindungsgemäße Mittel dadurch gekennzeichnet sind, daß die Metallsalze und/oder Metallkomplexe ausgewählt sind aus der Gruppe MnSO₄, Mn(II)-citrat, Mn(II)-stearat, Mn(II)-acetylacetonat, Mn(II)-[1-Hydroxyethan-1,1-diphosphonat], V₂O₅, V₂O₄, VO₂, TiOSO₄, K₂TiF₆, K₂ZrF₆, CoSO₄, Co(NO₃)₂, Ce(NO₃)₃.

Bei diesen Metallsalzen bzw. Metallkomplexen handelt es sich im allgemeinen um handelsübliche Substanzen, die zum Zwecke des Silberkorrosions-Schutzes ohne vorherige Reinigung in den erfindungsgemäßen Mitteln eingesetzt werden können.

So ist z.B. das aus der SO₃-Herstellung (Kontaktverfahren) bekannte Gemisch aus fünf- und vierwertigem Vanadium (V₂O₅, VO₂, V₂O₄) geeignet, ebenso wie das durch Verdünnen einer Ti(SO₄)₂-Lösung entstehende Titanylsulfat, TiOSO₄.

Die genannten Metallsalze und/oder Metallkomplexe sind in den erfindungsgemäßen Mitteln, vorzugsweise in einer Menge von 0,05 bis 6 Gew.-%, vorzugsweise 0,2 bis 2,5 Gew.%, bezogen auf das gesamte Mittel ohne den Behälter, enthalten

Ein weiteres wichtiges Kriterium zur Beurteilung eines maschinellen Geschirrspülmittels ist neben dessen Reinigungsleistung das optische Erscheinungsbild des trockenen Geschirrs nach erfolgter Reinigung. Eventuell auftretende Calciumcarbonat-Ablagerungen auf Geschirr oder im Maschineninnenraum können beispielsweise die Kundenzufriedenheit beeinträchtigen und haben damit ursächlichen Einfluß auf den wirtschaftlichen Erfolg eines derartigen Reinigungsmittels. Ein weiteres seit langem bestehendes Problem beim maschinellen Geschirrspülen ist die Korrosion von Glasspülgut, die sich in der Regel durch Auftreten von Trübungen, Schlieren und Kratzern, aber auch durch ein Irisieren der Glasoberfläche äußern kann. Die beobachteten Effekte beruhen dabei im wesentlichen auf zwei Vorgängen, zum einen dem Austritt von Alkali- und Erdalkaliionen aus dem Glas in Verbindung mit einer Hydrolyse des Silikat-Netzwerks, zum anderen in einer Ablagerung silikatischer Verbindungen auf der Glasoberfläche.

Die genannten Probleme können mit den erfindungsgemäßen Mitteln gelöst werden, wenn zusätzlich zu den vorstehend genannten zwingenden und gegebenenfalls optionalen Inhaltsstoffen bestimmte Glaskorrosionsinhibitoren in die Mittel inkorporiert werden. Bevorzugte erfindungsgemäße Mittel enthalten daher zusätzlich ein oder mehrere Magnesium- und/oder Zinksalze und/oder Magnesium- und/oder Zinkkomplexe.

Eine bevorzugte Klasse von Verbindungen, die zur Verhinderung der Glaskorrosion den erfindungsgemäßen Mitteln zugesetzt werden können, sind unlösliche Zinksalze. Diese können sich während des Geschirrspülvorgangs, an der Glasoberfläche anlagern und verhindern dort das in Lösung gehen von Metallionen aus dem Glasnetzwerk sowie die Hydrolyse der Silikate. Zusätzlich verhindern diese unlöslichen Zinksalze auch die Ablagerung von Silikat auf der Glasoberfläche, so daß das Glas vor den vorstehend geschilderten Folgen geschützt ist.

Unlösliche Zinksalze im Sinne dieser bevorzugten Ausführungsform sind Zinksalze, die eine Löslichkeit von maximal 10 Gramm Zinksalz pro Liter Wasser bei 20°C besitzen. Beispiele für erfindungsgemäß besonders bevorzugte unlösliche Zinksalze sind Zinksilikat, Zinkcarbonat, Zinkoxid, basisches Zinkcarbonat (Zn₂(OH)₂CO₃), Zinkhydroxid, Zinkoxalat, Zinkmonophosphat (Zn₃(PO₄)₂), und Zinkpyrophosphat (Zn₂(P₂O₇)).

Die genannten Zinkverbindungen werden in den erfindungsgemäßen Mitteln in Mengen eingesetzt, die einen Gehalt der Mittel an Zinkionen zwischen 0,02 und 10 Gew.-%, vorzugsweise zwischen 0,1 und 5,0 Gew.-% und insbesondere zwischen 0,2 und 1,0 Gew.-%, jeweils bezogen auf das Mittel ohne den Behälter, bewirken. Der exakte Gehalt der Mittel am Zinksalz bzw. den Zinksalzen ist naturgemäß abhängig von der Art der Zinksalze-je weniger löslich das eingesetzte Zinksalz ist, umso höher sollte dessen Konzentration in den erfindungsgemäßen Mitteln sein.

Eine weitere bevorzugte Klasse von Verbindungen sind Magnesium- und/oder Zinksalz(e) mindestens einer monomeren und/oder polymeren organischen Säure. Diese bewirken, daß auch bei wiederholter Benutzung die Oberflächen gläsernen Spülguts nicht korrosiv verändert, insbesondere keine Trübungen, Schlieren oder Kratzer aber auch kein frisieren der Glasoberflächen verursacht werden.

Obwohl erfindungsgemäß alle Magnesium- und/oder Zinksalz(e) monomerer und/oder polymerer organischer Säuren in den beanspruchten Mitteln enthalten sein können, werden doch, wie vorstehend beschrieben, die Magnesium- und/oder Zinksalze monomerer und/oder polymerer organischer Säuren aus den Gruppen der unverzweigten gesättigten oder ungesättigten Monocarbonsäuren, der verzweigten gesättigten oder ungesättigten Monocarbonsäuren, der gesättigten und ungesättigten Dicarbonsäuren, der aromatischen Mono-, Di- und Tricarbonsäuren, der Zuckersäuren, der Hydroxysäuren, der Oxosäuren, der Aminosäuren und/oder der polymeren Carbonsäuren bevorzugt. Innerhalb dieser Gruppen werden im Rahmen der vorliegenden Erfindung wiederum die in der Folge genannten Säuren bevorzugt:

Das Spektrum der erfindungsgemäß bevorzugten Zinksalze organischer Säuren, vorzugsweise organischer Carbonsäuren, reicht von Salzen, die in Wasser schwer oder nicht löslich sind, also eine Löslichkeit unterhalb 100 mg/L, vorzugsweise unterhalb 10 mg/L, insbesondere keine Löslichkeit aufweisen, bis zu solchen Salzen, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen (alle Löslichkeiten bei 20°C Wassertemperatur). Zu der ersten Gruppe von Zinksalzen gehören beispielsweise das Zinkcitrat, das Zinkoleat und das Zinkstearat, zu der Gruppe der löslichen Zinksalze gehören beispielsweise das Zinkformiat, das Zinkacetat, das Zinklactat und das Zinkgluconat:

In einer weiteren bevorzugten Ausführungsform der vorliegenden Erfindung enthalten die erfindungsgemäßen Mittel wenigstens ein Zinksalz, jedoch kein Magnesiumsalz einer organischen Säure, wobei es sich vorzugsweise um mindestens ein Zinksalz einer organischen Carbonsäure, besonders bevorzugt um ein Zinksalz aus der Gruppe Zinkstearat, Zinkoleat, Zinkgluconat, Zinkacetat, Zinklactat und/oder Zinkcitrat handelt. Auch Zinkricinoleat, Zinkabietat und Zinkoxalat sind bevorzugt.

Ein im Rahmen der vorliegenden Erfindung bevorzugtes Mittel enthält Zinksalz in Mengen von 0,1 bis 5 Gew.-%, vorzugsweise von 0,2 bis 4 Gew.-% und insbesondere von 0,4 bis 3 Gew.-%, bzw. Zink in oxidierter Form (berechnet als Zn²⁺) in Mengen von 0,01 bis 1 Gew.-%, vorzugsweise von 0,02 bis 0,5 Gew.-% und insbesondere von 0,04 bis 0,2 Gew.-%, jeweils bezogen auf das Mittel ohne den Behälter.

Ein weiterer bevorzugter Gegenstand der vorliegenden Anmeldung ist daher ein Duftabgabesystem, welches weitere Aktivsubstanzen, insbesondere Aktivsubstanzen aus der Gruppe der Parfümträger, Farbstoffe, antimikrobiellen Wirkstoffe, Germizide, Fungizide, Antioxidantien oder Korrosionsinhibitoren enthalten.

Die Erfindung ist nachstehend anhand der Zeichnung beispielhaft näher erläutert. Diese zeigt in:
Fig. 1 ein erfindungsgemäßes Duftabgabesystem im Querschnitt nach der Einfüllung der Partikel und vor dem Verschließen des Behälters,
Fig. 2 das Duftabgabesystem nach Figur 1 nach dem Verschließen des Behälters,
Fig. 3 einen Querschnitt durch den Behälter des Duftabgabesystems,
Fig. 4 den geschlossenen Behälter des Duftabgabesystems in perspektivischer Darstellung in einer Rückansicht und
Fig. 5 den Behälter nach Figur 4 ebenfalls in perspektivischer Darstellung in einer Vorderansicht.

Ein allgemein mit 1 bezeichnetes Duftabgabesystem weist einen allgemein mit 2 bezeichneten, im Wesentlichen rotationssymmetrischen Behälter mit einem Aufnahmeraum 3 und eine Vielzahl von im Aufnahmeraum 3 des Behälters 2 aufgenommenen Partikeln 4 zur Desodorierung und/oder Beduftung von geschlossenen Räumen, insbesondere den Innenräumen von Geschirrspülmaschinen, auf.

Die Partikel 4 weisen ein Trägermaterial, vorzugsweise aus einem Polymer, sowie wenigstens einen Duftstoff auf und haben bevorzugt einen Schmelz- oder Erweichungspunkt zwischen 30° und 150°C, insbesondere 75° und 80°C. Bevorzugte Materialien für die Partikel 4 sind ausführlich in der nicht vorveröffentlichten älteren deutschen Patentanmeldung 102 37 066.4 beschrieben, auf die zur Vermeidung von Wiederholungen ausdrücklich Bezug genommen und deren Offenbarung zum Gegenstand der Offenbarung dieser Anmeldung gemacht wird.

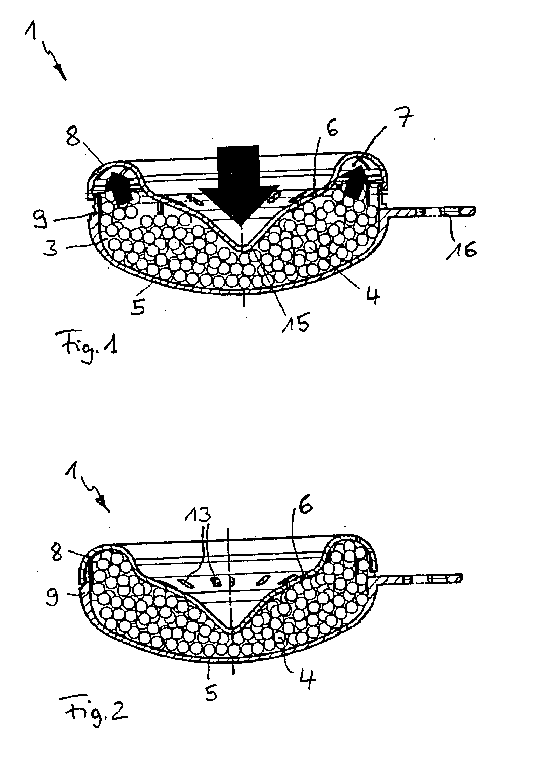

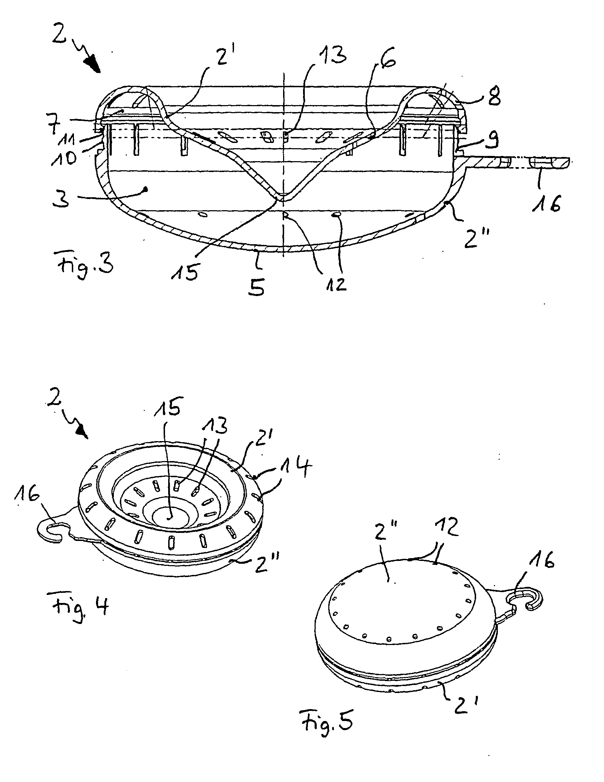

Wesentlich für das erfindungsgemäße Duftabgabesystem 1 ist die Gestaltung des Behälters 2. Wie am besten aus den Figuren 1 bis 3 hervorgeht, weist der Aufnahmeraum 3 des Behälters eine mondsichelartige Querschnittsform mit einer nach außen gewölbten Vorderwand 5 und einer nach innen gewölbten Rückwand 6 auf, wobei die beiden Endbereiche 7 der mondsichelartigen Querschnittsform des Aufnahmebereiches 3 abgerundet ausgebildet sind.

Dieser Behälter 2 kann einteilig ausgebildet sein und beispielsweise durch Blasformen hergestellt werden, es ist dann eine Einfüllöffnung für die Partikel 4 vorzusehen, die nach dem Einfüllen der Partikel 4 verschlossen wird.

Bevorzugt ist aber vorgesehen, wie dies in den Ausführungsbeispielen dargestellt ist, dass der Behälter 2 zweiteilig ausgebildet ist, wobei der eine Teil 2' die Rückwand 6 und der andere Teil 2" die Vorderwand 5 aufweist.

Der die Rückwand 6 aufweisende Teil 2' des Behälters 2 weist ferner einen wulstartigen Randbereich 8 auf, welcher mit einem stegartigen Randbereich 9 des anderen Teiles 2" verbindbar ist, wobei zur Verbindung bevorzugt eine Rastverbindung vorgesehen ist. So ist beim Ausführungsbeispiel am stegartigen Randbereich 9 eine Rastwulst 10 und innenseitig am wulstartigen Randbereich 8 eine Rastnase 11 vorgesehen.

Im Bereich der nach außen gewölbten Vorderwand 5 weist der Behälter 2 eine Mehrzahl von Öffnungen 12 auf, durch welche eine Emittierung der Duftstoffe der Partikel 4 vom Aufnahmeraum 3 nach außen möglich ist. Ferner sind im Bereich der Rückwand 6 und im Bereich des wulstartigen Randbereiches 8 eine Mehrzahl von schlitzförmigen Öffnungen 13 bzw. 14 vorgesehen.

Bei der dargestellten zweiteiligen Ausgestaltung des Behälters 2 ist ferner zur Vereinfachung des nachfolgend geschilderten Befüllvorganges vorgesehen, dass die nach innen gewölbte Rückwand 6 in ihrem mittleren Bereich 15 kegelförmig nach innen eingewölbt ist.

In bevorzugter Ausführung besteht das Duftabgabesystem 1 aus parfümierten Polymerpartikeln (z.B. Ethylen/Vinylacetat-Copolymer), eingeschlossen in einem perforierten Behälter 2 (z.B. aus Propylen). Die Polymerpartikel können beispielsweise von Elvax® 265 der Firma Dupont mit einem Schmelzpunkt von 75°C gebildet sein, welche bei Raumtemperatur etwa 25 bis 30 % Parfümöl aufnehmen können. Dieses Granulat ist ein gängiger Rohstoff für die kunststoffverarbeitende Industrie und leicht verfügbar. Die Tatsache, dass diese Partikel bei Niedrigtemperatur mit Parfümöl beladen werden können und ein Verbrennen des Parfümöles vermieden werden kann, macht diese Partikel besonders geeignet für ihre Anwendung in einem erfindungsgemäßen Duftabgabesystem. Der Radius der Partikel 4 beträgt etwa 1,5 bis 3 mm und ermöglicht eine schnelle wirtschaftliche Beladung mit Parfümöl.

Die beiden Teile 2', 2" des Behälters 2 sind bevorzugt im Spritzgießverfahren aus Polypropylen hergestellt, eines der beiden Teile 2', 2" weist an seiner Außenseite einen Haken 16 zum Einhängen an einen Korb einer Spülmaschine oder dergl. auf. Die Behälterteile 2', 2" können auch auf andere Weise hergestellt werden oder aus anderen Kunststoffmaterialien bestehen, wie dies im Einzelnen ausführlich in der älteren, nicht vorveröffentlichten deutschen Patentanmeldung 102 37 066.4 dargelegt ist, worauf ausdrücklich verwiesen wird.

Während die Öffnungen 12 in der Vorderwand 5 in erster Linie zur Emittierung des Parfümöles von den Partikeln 4 nach außen, beispielsweise in.den Innenraum einer Geschirrspülmaschine, dienen, sind die vorzugsweise schlitzförmigen Öffnungen 13, 14 an der Rückwand 6 des Behälters dazu vorgesehen, um ein Ein- und Auslaufen von Wasser beim Spülvorgang zu ermöglichen, um die Duftabgabe zu verbessern.

Der Aufnahmeraum 3 des Behälters 2 weist beispielsweise ein Volumen von 40 ml auf, mit einer Höhe und daraus folgendernd einer Schichtdicke der Partikel 4 zwischen etwa 10 bis 12 mm. Dieser Aufnahmeraum 3 wird, wie in den Figuren 1 und 2 dargestellt, vollständig mit Partikeln 4 gefüllt. In der Befüllposition (Figur 1) bildet der die Vorderwand 5 aufweisende Teil 2" die eigentliche Aufnahme des Behälters 2, während der die Rückwand 6 aufweisende Teil 2' eine Art Deckel darstellt.

Die benötigte Menge an Partikeln 4 wird zunächst in den Teil 2" eingefüllt, so dass der Füllstand etwas unterhalb des oberen Randes des stegartigen Randbereiches 9 verbleibt. Anschließend wird der Teil 2' aufgelegt und abgesenkt (großer Pfeil in Figur 1). Etwa das erste Drittel der nach innen gewölbten Rückwand 6 mit kegelförmigem Mittelbereich 15 taucht dabei in die Partikel 4 ein und verdrängt teilweise Partikel 4 zur Peripherie des Behälters 2 und nach oben. Bevor die Partikel 4 den oberen Rand erreichen, erreicht der wulstartige Randbereich 8 des Behälterteiles 2' den stegartigen Randbereich 9 des Teiles 2" und die Partikel 4 können nicht aus dem Behälter 2 herausfallen. Das weitere Absenken des Teiles 2' (Figur 1 großer Pfeil) bewirkt das Eintauchen des weiteren Teiles des kegelförmigen Mittelbereiches 15, was dazu führt, dass Partikel aus dem Peripheriebereich nach oben in den noch freien Bereich des Aufnahmeraumes 3 verdrängt werden (kleine Pfeile in Figur 1), wodurch auch dieser Bereich des Aufnahmeraumes 3 vollständig befüllt wird. Dabei rasten beide Teile 2', 2" aufgrund der Rastverbindung 9, 10 ineinander ein und der Behälter 2 ist dauerhaft geschlossen. Bei einer genauen Dosiermenge an Partikeln 4 ist dadurch der Behälter 2 vollständig befüllt, wie in Figur 2 dargestellt.

Die Partikel 4 sind dadurch nicht lose im Behälter 2 angeordnet und bilden nach dem ersten Spülvorgang in einer Spülmaschine durch Erreichen ihrer Erwärmungstemperatur ein mondsichelartiges Konglomerat mit einer Schichtdicke von 10 bis 12 mm, was die exponierte Oberfläche der Partikel 4 reduziert und eine lange Funktionalität des Systems ermöglicht. Wenn demgegenüber die Partikel 4 frei beweglich im Behälter 2 wären, wäre die Funktionsdauer des Duftabgabesystems nur von der Parfümölmenge abhängig, die in jedem Partikel 4 enthalten ist, die Anzahl der Partikel 4 würde nur die Duftintensität beeinflussen.

Durch die erfindungsgemäße Gestaltung des Duftabgabesystems wird also die exponierte Oberfläche der Partikel 4 reduziert, so dass nicht alle Partikel auf einmal ihren Duft abgeben können, die Duftintensität wird somit von der Partikelmenge an der Oberfläche des Konglomerates im direkten Kontakt mit der Luft bestimmt, nicht jedoch von allen Partikeln. Das Parfümöl aus den inneren Partikeln migriert progressiv zur Oberfläche des Konglomerates, die inneren Partikel spielen somit eine Depotfunktion für das Duftabgabesystem und ermöglichen dadurch eine wesentlich längere Funktionsdauer des Systems. Es hat sich herausgestellt, dass mit einem derartigen Duftabgabesystem in einer Geschirrspülmaschine durchaus 50 Spülvorgänge bei ca. 3 bis 4 Spülgängen pro Woche ohne Funktionsbeeinträchtigung möglich sind.

Durch die besondere Gestaltung des Behälters 2 des Duftabgabesystems 1 läßt sich ein besonders günstiges Verhältnis der Gesamtoberfläche sämtlicher Partikel 4 im Ausgangszustand (vor ihrer erstmaligen Erwärmung beispielsweise bei einem Spülvorgang) zur Gesamtoberfläche des Aufnahmeraumes erreichen. So ergibt sich bei einem Volumen des Aufnahmeraumes 3 von 40 ml eine Gesamtpartikeloberfläche von ca. 250 cm², unter der Annahme, dass die Partikel 4 etwa kugelförmig sind und einen durchschnittlichen Radius von 2 mm aufweisen. Die Innenfläche des Aufnahmeraumes 3 des Gehäuses beträgt etwa 88,5 cm², so dass angenommen werden kann, dass das Partikelkonglomerat im geschlossenen Behälter insgesamt ebenfalls eine Oberfläche von 88,5 cm² hat. Daraus ergibt sich ein Verhältnis zwischen der Gesamtoberfläche sämtlicher Partikel im Ausgangszustand zur Gesamtoberfläche des Aufnahmeraumes von 1 : 0,353 (Annäherungswert) als Optimalwert für das Duftabgabesystem, um eine effektive-Duftabgabe bei geringem Behältervolumen zu gewährleisten.

Wenn demgegenüber die Partikel in einem kugelförmigen Gehäuse mit dem gleichen Volumen (40 ml) eingeschlossen wären, würde die Innenoberfläche dieser Kugel 56,8 cm² betragen. Daraus ergäbe sich ein Verhältnis von 1 : 0,227. Eine solche Ausführungsform des Behälters 2 hat sich jedoch als ungünstig erwiesen, weil die duftende Oberfläche der Partikel 4 zu klein ist und dadurch die Duftintensität zu stark abnimmt.

Die vorbeschriebenen Verfahren zur Konfektionierung der Behälter erfindungsgemäßer Mittel werden sich in aller Regel ebenso nach optischen Gesichtspunkten wie dem letztendlichen Bestimmungszweck dieser Mittel richten. Die erfindungsgemäßen Mittel können beispielsweise neben den duftstoffhaltigen Partikeln weitere Aktivsubstanzen enthalten. Diese Aktivsubstanzen können innerhalb des Behälters in einer Mischung bzw. einem Gemenge mit den duftstoffhaltigen Partikeln aber auch getrennt von diesen Partikeln konfektioniert werden. Die Aktivsubstanzen können aber auch in den Behälter eingearbeitet sein. Dabei können diese optionalen zusätzlichen Aktivsubstanzen sowohl in Form einer Einzeldosierung, beispielsweise für die einmalige Desinfektion einer Geschirrspülmaschine, aber auch in Form einer Mehrfachdosierung eingesetzt werden.

Zusätzlich zu den vorgenannten Aktivsubstanzen können die erfindungsgemäßen Mittel, insbesondere Mittel für den Einsatz in Geschirrspülmaschinen, Textilwaschmaschinen oder -trockern, selbstverständlich alle üblicherweise in Mitteln für die Textil- oder Geschirreinigung bzw. die Textil- oder Geschirrpflege enthaltenen Aktivsubstanzen aufweisen, wobei Substanzen aus der Gruppe der Bleichmittel, Bleichaktivatoren, Polymere, Gerüststoffe, Tenside, Enzyme, Elektrolyte, pH-Stellmittel, Duftstoffe, Parfümträger, Farbstoffe, Hydrotrope, Schauminhibitoren, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Korrosionsinhibitoren, Antistatika, Phobier- und Imprägniermittel, Quell- und Schiebefestmittel, nichtwässrigen Lösungsmittel, Weichspüler, Proteinhydrolysate, sowie UV-Absorber besonders bevorzugt sind. Derartige Kombinationsprodukte eignen sich dann neben der wiederholten Beduftung auch zur ein- oder mehrmaligen Pflege oder Reinigung von Textilien oder Geschirr.

Als wichtige Bestandteile von Wasch- oder Reinigungsmitteln können in den erfindungsgemäßen Mitteln neben anderen Bestandteilen Bleichmittel und Bleichaktivatoren enthalten sein. Unter den als Bleichmittel dienenden, in Wasser H₂O₂ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthajoiminopersäure oder Diperdodecandisäure. Reinigungsmittelformkörper für das maschinelle Geschirrspülen können auch Bleichmittel aus der Gruppe der organischen Bleichmittel enthalten. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesium-monoperphthalat, (b) die aliphatischen oder substituiert aliphatischen Peroxysäuren, wie Peroxylaurinsäure, Peroxystearinsäure, ε-Phthalimidoperoxycapronsäure [Phthaloiminoperoxyhexansäure (PAP)], o-Carboxybenzamidoperoxycapronsäure, N-nonenylamidoperadipinsäure und N-nonenylamidopersuccinate, und (c) aliphatische und araliphatische Peroxydicarbonsäuren, wie 1,12-Diperoxycarbonsäure, 1,9-Diperoxyazelainsäure, Diperocysebacinsäure, Diperoxybrassylsäure, die Diperoxyphthatsäuren, 2-Decytdiperoxybutan-1,4-disäure, N,N-Terephthaloyl-di(6-aminopercapronsäure) können eingesetzt werden.

Werden die erfindungsgemäßen Mittel in Kombination mit maschinellen Geschirrspülmitteln eingesetzt, so können diese Bleichaktivatoren enthalten, um beim Reinigen bei Temperaturen von 60 °C und darunter eine verbesserte Bleichwirkung zu erreichen. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Weitere im Rahmen der vorliegenden Anmeldung bevorzugt eingesetzte Bleichaktivatoren sind Verbindungen aus der Gruppe der kationischen Nitrile, insbesondere kationische Nitrite der Formel 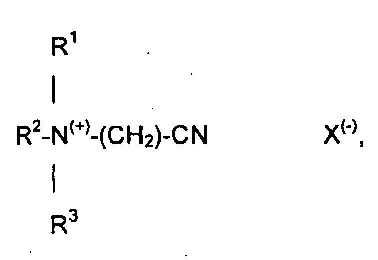 in der R¹ für-H, -CH₃, einen C₂₋₂₄-Alkyl- oder -Alkenylrest, einen substituierten C₂₋₂₄-Alkyl- oder -Alkenylrest mit mindestens einem Substituenten aus der Gruppe -Cl, -Br, -OH, -NH₂, -CN, einen Alkyl- oder Alkenylarylrest mit einer C₁₋₂₄-Alkylgruppe, oder für einen substituierten Alkyl- oder Alkenylarylrest mit einer C₁₋₂₄-Alkylgruppe und mindestens einem weiteren Substituenten am aromatischen Ring steht, R² und R³ unabhängig voneinander ausgewählt sind aus -CH₂-CN, -CH₃, -CH₂-CH₃, -CH₂-CH₂-CH₃, -CH(CH₃)-CH₃, -CH₂-OH, -CH₂-CH₂-OH, -CH(OH)-CH₃, -CH₂-CH₂-CH₂-OH, -CH₂-CH(OH)-CH₃, -CH(OH)-CH₂-CH₃, -(CH₂CH₂-O)ₙH mit n = 1, 2, 3, 4, 5 oder 6 und X ein Anion ist.

In besonders bevorzugten erfindungsgemäßen Mitteln ist ein kationisches Nitril der Formel 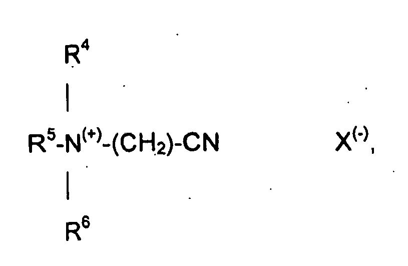 enthalten, in der R⁴, R⁵ und R⁶ unabhängig voneinander ausgewählt sind aus =CH₃, -CH₂-CH₃, -CH₂-CH₂-CH₃, -CH(CH₃)-CH₃, wobei R⁴ zusätzlich auch -H sein kann und X ein Anion ist, wobei vorzugsweise R⁵ = R⁶ = -CH₃ und insbesondere R⁴ = R⁵ = R⁶ = -CH₃ gilt und Verbindungen der Formeln (CH₃)₃N⁽⁺⁾CH₂-CN X⁻, (CH₃CH₂)₃N⁽⁺⁾CH₂-CN X⁻, (CH₃CH₂CH₂)₃N⁽⁺⁾CH₂-CN X⁻, (CH₃CH(CH₃))₃N⁽⁺⁾CH₂-CN X⁻, oder (HO-CH₂-CH₂)₃N⁽⁺⁾CH₂-CN X⁻ besonders bevorzugt sind, wobei aus der Gruppe dieser Substanzen wiederum das kationische . Nitril der Formel (CH₃)₃N⁽⁺⁾CH₂-CN X⁻, in welcher X⁻ für ein Anion steht, das aus der Gruppe Chlorid, Bromid, lodid, Hydrogensulfat, Methosulfat, p-Toluolsulfonat (Tosylat) oder Xylolsulfonat ausgewählt ist, besonders bevorzugt wird.

Zusätzlich zu den konventionellen Bleichaktivatoren oder an deren Stelle können auch sogenannte Bleichkatalysatoren in die Mittel eingearbeitet werden. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Neben den genannten Inhaltsstoffen Bleichmittel und Bleichaktivator sind Gerüststoffe weitere wichtige Inhaltsstoffe von Wasch- oder Reinigungsmitteln. In den erfindungsgemäßen Mitteln können dabei alle üblicherweise in diesen Mitteln eingesetzten Gerüststoffe enthalten sein, insbesondere also Zeolithe, Silikate, Carbonate, organische Cobuilder und - wo keine ökologischen Vorurteile gegen ihren Einsatz bestehen - auch die Phosphate.

Geeignete kristalline, schichtförmige Natriumsilikate besitzen die allgemeine Formel NaMSiₓO₂ₓ₊₁ H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl β-als auch δ-Natriumdisilikate Na₂Si₂O₅ yH₂O bevorzugt.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silikate weisen ebenfalls eine Löseverzögerung gegenüber den herkömmlichen Wassergläsern aufweisen auf. Insbesondere bevorzugt sind verdichtete/kompaktierte amorphe Silikate, compoundierte amorphe Silikate und übertrocknete röntgenamorphe Silikate.

Der einsetzbare feinkristalline, synthetische und gebundenes Wasser enthaltende Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O (1-n)K₂O · Al₂O₃ · (2 - 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate. Zur Vermeidung von Wiederholungen wird für eine ausführliche Beschreibung dieser Phosphate auf die obigen Ausführungen verwiesen.

Brauchbare organische Gerüstsubstanzen sind beispielsweise die in Form ihrer Alkali- und insbesondere Natriumsalze einsetzbaren Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Als weitere Bestandteile können Alkaliträger zugegen sein. Als Alkaliträger gelten Alkalimetallhydroxide, Alkalimetallcarbonate, Alkalimetallhydrogencarbonate, Alkalimetallsesquicarbonate, Alkalisilikate, Alkalimetasilikate, und Mischungen der vorgenannten Stoffe, wobei im Sinne dieser Erfindung bevorzugt die Alkalicarbonate, insbesondere Natriumcarbonat, Natriumhydrogencarbonat oder Natriumsesquicarbonat eingesetzt werden.

Werden die erfindungsgemäßen Mittel beim maschinellen Geschirrspülen eingesetzt, so sind wasserlösliche Builder bevorzugt, da sie auf Geschirr und harten Oberflächen in der Regel weniger dazu tendieren, unlösliche Rückstände zu bilden. Übliche Builder sind die niedermolekularen Polycarbonsäuren und ihre Salze, die homopolymeren und copolymeren Polycarbonsäuren und ihre Salze; die Carbonate, Phosphate und Silikate. Bevorzugt werden zur Herstellung von Tabletten für das maschinelle Geschirrspülen Trinatriumcitrat und/oder Pentanatriumtripolyphosphat und/oder Natriumcarbonat und/oder Natriumbicarbonat und/oder Gluconate und/oder silikatische Builder aus der Klasse der Disilikate und/oder Metasilikate eingesetzt. Besonders bevorzugt ist ein Buildersystem enthaltend eine Mischung aus Tripolyphosphat und Natriumcarbonat. Ebenfalls besonders bevorzugt ist ein Buildersystem, das eine Mischung aus Tripolyphosphat und Natriumcarbonat und Natriumdisilikat enthält.

Als organische Cobuilder können in den Reinigungsmitteln im Rahmen der vorliegenden Erfindung insbesondere Polycarboxylate / Polycarbonsäuren, polymere Polycarboxylate, Asparaginsäure, Polyacetale, Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Methylglycindiessigsäure, Zuckersäuren und Mischungen aus diesen.

Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 1000 bis 20000 g/mol aufweisen. Aufgrund ihrer überlegenen Löslichkeit können aus dieser Gruppe wiederum die kurzkettigen Polyacrylate, die Molmassen von 1000 bis 10000 g/mol, und besonders bevorzugt von 1200 bis 4000 g/mol, aufweisen, bevorzugt sein.

Besonders bevorzugt werden in den erfindungsgemäßen Mitteln sowohl Polyacrylate als auch Copolymere aus ungesättigten Carbonsäuren, Sulfonsäuregrupperi-haltigen Monomeren sowie gegebenenfalls weiteren ionischen oder nichtionogenen Monomeren eingesetzt. Die Sulfonsäuregruppen-haltigen Copolymere werden in der Folge ausführlich beschrieben.

Es lassen sich aber auch erfindungsgemäße Produkte, welche als sogenannte "3in1"-Produkte die herkömmlichen Reiniger, Klarspüler und eine Salzersatzfunktion in sich vereinen, bereitstellen. Hierzu sind erfindungsgemäße maschinelle Geschirrspülmittel bevorzugt, die zusätzlich 0,1 bis 70 Gew.-% an Copolymeren aus
i) ungesättigten Carbonsäuren
ii) Sulfonsäuregruppen-haltigen Monomeren
iii) gegebenenfalls weiteren ionischen oder nichtionogenen Monomeren
enthalten.

Diese Copolymere bewirken, daß die mit solchen Mitteln behandelten Geschirrteile bei nachfolgenden Reinigungsvorgängen deutlich sauberer werden, als Geschirrteile, die mit herkömmlichen Mitteln gespült wurden.

Als zusätzlicher positiver Effekt tritt eine Verkürzung der Trocknungszeit der mit dem Reinigungsmittel behandelten Geschirrteile auf, d.h. der Verbraucher kann nach dem Ablauf des Reinigungsprogramms das Geschirr früher aus der Maschine nehmen und wiederbenutzen. Unter Trocknungszeit wird im Rahmen der erfindungsgemäßen Lehre im allgemeinen die wortsinngemäße Bedeutung verstanden, also die Zeit, die verstreicht, bis eine in einer Geschirrspülmaschine behandelte Geschirroberfläche getrocknet ist, im besonderen aber die Zeit, die verstreicht, bis 90 % einer mit einem Reinigungs- oder Klarspülmittel in konzentrierter oder verdünnter Form behandelten Oberfläche getrocknet ist.

Im Rahmen der vorliegenden Erfindung sind ungesättigte Carbonsäuren der Formel I als Monomer bevorzugt,

R¹(R²)C=C(R³)COOH (I),

in der R¹ bis R³ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Unter den ungesättigten Carbonsäuren, die sich durch die Formel I beschreiben lassen, sind insbesondere Acrylsäure (R¹ = R² = R³ = H), Methacrylsäure (R¹ = R² = H; R³ = CH₃) und/oder Maleinsäure (R¹ = COOH; R² = R³ = H) bevorzugt.

Bei den Sulfonsäuregruppen-haltigen Monomeren sind solche der Formel II bevorzugt,

R⁵(R⁶)C=C(R⁷)-X-SO₃H (II),

in der R⁵ bis R⁷ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für-COOH oder-COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂- und -C(O)-NH-CH(CH₂CH₃)-.

Unter diesen Monomeren bevorzugt sind solche der Formeln IIa, IIb und/oder IIc,

H₂C=CH-X-SO₃H (IIa),

H₂C=C(CH₃)-X-SO₃H (IIb),

HO₃S-X-(R⁶)C=C(R⁷)-X-SO₃H (IIc),

in denen R⁶ und R⁷ unabhängig voneinander ausgewählt sind aus -H, -CH₃, -CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂- und -C(O)-NH-CH(CH₂CH₃)-.

Besonders bevorzugte Sulfonsäuregruppen-haltige. Monomere sind dabei 1-Acrylamido-1-propansulfonsäure (X = -C(O)NH-CH(CH₂CH₃) in Formel IIa), 2-Acrylamido-2-propansulfonsäure (X = -C(O)NH-C(CH₃)₂ in Formel IIa), 2-Acrylamido-2-methyl-1-propansulfonsäure (X = -C(O)NH-CH(CH₃)CH₂- in Formel IIa), 2-Methacrylamido-2-methyl-1-propansutfonsäure (X = -C(O)NH-CH(CH₃)CH₂- in Formel IIb), 3-Methacrylamido-2-hydroxy-propansulfonsäure (X = -C(O)NH-CH₂CH(OH)CH₂- in Formel IIb), Allylsulfonsäure (X = CH₂ in Formel IIa), Methallylsulfonsäure (X = CH₂ in Formel IIb), Allyloxybenzolsulfonsäure (X = -CH₂-O-C₆H₄- in Formel IIa), Methallyloxybenzolsulfonsäure (X = -CH₂-O-C₆H₄- in Formel Ilb), 2-Hydroxy-3-(2-propenyloxy)propansulfonsäure, 2-Methyl-2-propen1-sulfonsäure (X = CH₂ in Formel IIb), Styrolsulfonsäure (X = C₆H₄ in Formel Ila), Vinylsulfonsäure (X nicht vorhanden in Formel Ila), 3-Sulfopropylacrylat (X = -C(O)NH-CH₂CH₂CH₂- in Formel IIa), 3-Sulfopropylmethacrylat (X = -C(O)NH-CH₂CH₂CH₂- in Formel IIb), Sulfomethacrylamid (X = -C(O)NH- in Formel Ilb), Sulfomethylmethacrylamid (X = -C(O)NH-CH₂- in Formel IIb) sowie wasserlösliche Salze der genannten Säuren.

Als weitere ionische oder nichtionogene Monomere kommen insbesondere ethylenisch ungesättigte Verbindungen in Betracht. Vorzugsweise beträgt der Gehalt der erfindungsgemäß eingesetzten Polymere an Monomeren der Gruppe iii) weniger als 20 Gew.-%, bezogen auf das Polymer. Besonders bevorzugt zu verwendende Polymere bestehen lediglich aus Monomeren der Gruppen i) und ii).

Zusammenfassend sind Copolymere aus
i) ungesättigten Carbonsäuren der Formel I.

   R¹(R²)C=C(R³)COOH (I),

   in der R¹ bis R³ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist,
ii) Sulfonsäuregruppen-haltigen Monomeren der Formel II

   R⁵(R⁶)C=C(R⁷)-X-SO₃H (II),

   in der R⁵ bis R⁷ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂- und -C(O)-NH-CH(CH₂CH₃)-
iii) gegebenenfalls weiteren ionischen oder nichtionogenen Monomeren
besonders bevorzugt.

Besonders bevorzugte Copolymere bestehen aus
i) einer oder mehrerer ungesättigter Carbonsäuren aus der Gruppe Acrylsäure, Methacrylsäure und/oder Maleinsäure
ii) einem oder mehreren Sulfonsäuregruppen-haltigen Monomeren der Formeln IIa, IIb und/oder IIc:

   H₂C=CH-X-SO₃H (IIa),

   H₂C=C(CH₃)-X-SO₃H (IIb),

   HO₃S-X-(R⁶)C=C(R⁷)-X-SO₃H (IIc),

   in der R⁶ und R⁷ unabhängig voneinander ausgewählt sind aus -H, -CH₃, -CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂- und -C(O)-NH-CH(CH₂CH₃)-
iii) gegebenenfalls weiteren ionischen oder nichtionogenen Monomeren.

Die in den Mitteln enthaltenen Copolymere können die Monomere aus den Gruppen i) und ii) sowie gegebenenfalls iii) in variierenden Mengen enthalten, wobei sämtliche Vertreter aus der Gruppe i) mit sämtlichen Vertretern aus der Gruppe ii) und sämtlichen Vertretern aus der Gruppe iii) kombiniert werden können. Besonders bevorzugte Polymere weisen bestimmte Struktureinheiten auf, die nachfolgend beschrieben werden.

So sind beispielsweise erfindungsgemäße Mittel bevorzugt, die dadurch gekennzeichnet sind, daß sie ein oder mehrere Copolymere enthalten,' die Struktureinheiten der Formel III

-[CH₂-CHCOOH]ₘ-[CH₂-CHC(O)-Y-SO₃H]ₚ- (III),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH2)ₙ- mit n = 0 bis 4, für -O-(C₆H₄)-, für -NH-C(CH₃)₂- oder -NH-CH(CH₂CH₃)- steht, bevorzugt sind.

Diese Polymere werden durch Copolymerisation von Acrylsäure mit einem Sulfonsäuregruppen-haltigen Acrylsäurederivat hergestellt. Copolymerisiert man das Sulfonsäuregruppen-haltige Acrylsäurederivat mit Methacrylsäure, gelangt man zu einem anderen Polymer, dessen Einsatz in den erfindungsgemäßen Mitteln ebenfalls bevorzugt und dadurch gekennzeichnet ist, daß die Mittel ein oder mehrere Copolymere enthalten, die Struktureinheiten der Formel IV

-[CH₂-C(CH₃)COOH]ₘ-[CH₂-CHC(O)-Y-SO₃H]ₚ- (IV),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind.

Völlig analog lassen sich Acrylsäure und/oder Methacrylsäure auch mit Sulfonsäuregruppen-haltigen Methacrylsäurederivaten copolymerisieren, wodurch die Struktureinheiten im Molekül verändert werden. So sind erfindungsgemäße Mittel, die ein oder mehrere Copolymere enthalten, welche Struktureinheiten der Formel V

-[CH₂-CHCOOH]ₘ-[CH₂-C(CH₃)C(O)-Y-SO₃H]ₚ- (V),

enthalten, in der m und p jeweils für eine ganze natürliche Zahl zwischen 1 und 2000 sowie Y für eine Spacergruppe steht; die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind, ebenfalls eine bevorzugte Ausführungsform der vorliegenden Erfindung, genau wie auch Mittel bevorzugt sind, die dadurch gekennzeichnet sind, daß sie ein oder mehrere Copolymere enthalten, die Struktureinheiten der Formel VI

-[CH₂-C(CH₃)COOH]ₘ-[CH₂-C(CH₃)C(O)-Y-SO₃H]ₚ- (VI),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit-n = 0 bis 4, für -O-(C₆H₄)-, für -NH-C(CH₃)₂- oder -NH-CH(CH₂CH₃)- steht, bevorzugt sind.

Anstelle von Acrylsäure und/oder Methacrylsäure bzw. in Ergänzung hierzu kann auch Maleinsäure als besonders bevorzugtes Monomer aus der Gruppe i) eingesetzt werden. Man gelangt auf diese Weise zu erfindungsgemäß bevorzugten Mitteln, die dadurch gekennzeichnet sind, daß sie ein oder mehrere Copolymere enthalten, die Struktureinheiten der Formel VII

-[HOOCCH-CHCOOH]ₘ-[CH₂-CHC(O)-Y-SO₃H]ₚ- (VII),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind und zu Mitteln, welche dadurch gekennzeichnet sind, daß sie ein oder mehrere Copolymere enthalten, die Struktureinheiten der Formel VIII

-[HOOCCH-CHCOOH]ₘ-[CH₂-C(CH₃)C(O)O-Y-SO₃H]ₚ- (VIII),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Köhlehstoffatomen, wobei Spacergruppen, in denen Y für -O-(CH₂)- mit n = 0 bis 4, für -O-(C₆H₄)-, für -NH-C(CH₃)₂- oder -NH-CH(CH₂CH₃)- steht, bevorzugt sind.

Zusammenfassend sind erfindungsgemäße maschinelle Geschirrspülmittel bevorzugt, die als Inhaltsstoff b) ein oder mehrere Copolymere enthält, die Struktureinheiten der Formeln III und/oder IV und/oder V und/oder VI und/oder VII und/oder VIII

-[CH₂-CHCOOH]ₘ-[CH₂-CHC(O)-Y-SO₃H]ₚ- (III),

-[CH₂-C(CH₃)COOH]ₘ-[CH₂-CHC(O)-Y-SO₃H]ₚ- (IV),

-[CH₂-CHCOOH]ₘ-[CH₂-C(CH₃)C(O)-Y-SO₃H]ₚ- (V),

-[CH₂-C(CH₃)COOH]ₘ-[CH₂-C(CH₃)C(O)-Y-SO₃H]ₚ- (VI),

-[HOOCCH-CHCOOH]ₘ-[CH₂-CHC(O)-Y-SO₃H]ₚ- (VII),

-[HOOCCH-CHCOOH]ₘ-[CH₂-C(CH₃)C(O)O-Y-SO₃H]ₚ- (VIII),

enthalten, in denen m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind.

In den Polymeren können die Sulfonsäuregruppen ganz oder teilweise in neutralisierter Form vorliegen, d.h. daß das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Entsprechende Mittel, die dadurch gekennzeichnet sind, daß die Sulfonsäuregruppen im Copolymer teil- oder vollneutralisiert vorliegen, sind erfindungsgemäß bevorzugt.

Die Monomerenverteilung der in den erfindungsgemäßen Mitteln eingesetzten Copolymeren beträgt bei Copolymeren, die nur Monomere aus den Gruppen i) und ii) enthalten, vorzugsweise jeweils 5 bis 95 Gew.-% i) bzw. ii), besonders bevorzugt 50 bis 90 Gew.-% Monomer aus der Gruppe i) und 10 bis 50 Gew.-% Monomer aus der Gruppe ii), jeweils bezogen auf das Polymer.

Bei Terpolymeren sind solche besonders bevorzugt, die 20 bis 85 Gew.-% Monomer aus der Gruppe i), 10 bis 60 Gew.-% Monomer aus der Gruppe ii) sowie 5 bis 30 Gew.-% Monomer aus der Gruppe iii) enthalten.

Die Molmasse der in den erfindungsgemäßen Mitteln eingesetzten Polymere kann variiert werden, um die Eigenschaften der Polymere dem gewünschten Verwendungszweck anzupassen. Bevorzugte maschinelle Geschirrspülmittel sind dadurch gekennzeichnet, daß die Copolymere Molmassen von 2000 bis 200.000 gmol⁻¹, vorzugsweise von 4000 bis 25.000 gmol⁻¹ und insbesondere von 5000 bis 15.000 gmol⁻¹ aufweisen.

Der Gehalt an einem oder mehreren Copolymeren in den erfindungsgemäßen Mitteln kann je nach Anwendungszweck und gewünschter Produktleistung varieren, wobei bevorzugte erfindungsgemäße maschinelle Geschirrspülmittel dadurch gekennzeichnet sind, daß sie das bzw. die Copolymer(e) in Mengen von 0,25 bis 50 Gew.-%, vorzugsweise von 0,5 bis 35 Gew.-%, besonders bevorzugt von 0,75 bis 20 Gew.-% und insbesondere von 1 bis 15 Gew.-% enthalten.

Wie bereits weiter oben erwähnt, werden in den erfindungsgemäßen Mitteln besonders bevorzugt sowohl Polyacrylate als auch die vorstehend beschriebenen Copolymere aus ungesättigten Carbonsäuren, Sulfonsäuregruppen-haltigen Monomeren sowie gegebenenfalls weiteren ionischen oder nichtionogenen Monomeren eingesetzt. Die Polyacrylate wurden dabei weiter oben ausführlich beschrieben. Besonders bevorzugt sind Kombinationen aus den vorstehend beschriebenen Sulfonsäuregruppen-haltigen Copolymeren mit Polyacrylaten niedriger Molmasse, beispielsweise im Bereich zwischen 1000 und 4000 Dalton. Solche Polyacrylate sind kommerziell unter dem Handelsnamen Sokalan^{®} PA15 bzw. Sokalan^{®} PA25 (BASF) erhältlich.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 100000 g/mol, vorzugsweise 20000 bis 90000 g/mol und insbesondere 30000 bis 80000 g/mol.

Die (co-)polymeren Polycarboxylate können entweder als Pulver oder als wäßrige Lösung eingesetzt werden. Der Gehalt der Mittel an (co-)polymeren Polycarboxylaten beträgt vorzugsweise 0,5 bis 20 Gew.-%, insbesondere 3 bis 10 Gew.-%.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsäure sowie Zucker-Derivate enthalten.

Weitere bevorzugte Copolymere weisen als Monomere vorzugsweise Acrolein und Acrylsäure/Acrylsäuresalze bzw. Acrolein und Vinylacetat auf.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.-%.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüber hinaus können alle Verbindungen, die in der Lage sind, Komplexe mit Erdalkaliionen auszubilden, als Cobuilder eingesetzt werden.

Bevorzugte Mittel enthalten im Rahmen der vorliegenden Anmeldung ein oder mehrere Tensid(e) aus den Gruppen der anionischen, nichtionischen, kationischen und/oder amphoteren Tenside.

Als Aniontenside in Säureform werden bevorzugt ein oder mehrere Stoffe aus der Gruppe der Carbonsäuren, der Schwefelsäurehalbester und der Sulfonsäuren, vorzugsweise aus der Gruppe der Fettsäuren, der Fettalkylschwefelsäuren und der Alkylarylsulfonsäuren, eingesetzt. Um ausreichende oberflächenaktive Eigenschaften aufzuweisen, sollten die genannten Verbindungen dabei über längerkettige Kohlenwasserstoffreste verfügen, also im Alkyl- oder Alkenylrest mindestens 6 C-Atome aufweisen. Üblicherweise liegen die C-Kettenverteilungen der Aniontenside im Bereich von 6 bis 40, vorzugsweise 8 bis 30 und insbesondere 12 bis 22 Kohlenstoffatome.

Carbonsäuren, die in Form ihrer Alkalimetallsalze als Seifen in Wasch- und Reinigungsmitteln Verwendung finden, werden technisch größtenteils aus nativen Fetten und Ölen durch Hydrolyse gewonnen. Während die bereits im vergangenen Jahrhundert durchgeführte alkalische Verseifung direkt zu den Alkalisalzen (Seifen) führte, wird heute großtechnisch zur Spaltung nur Wasser eingesetzt, das die Fette in Glycerin und die freien Fettsäuren spaltet. Großtechnisch angewendete Verfahren sind beispielsweise die Spaltung im Autoklaven oder die kontinuierliche Hochdruckspaltung. Im Rahmen der vorliegenden Erfindung als Aniontensid in Säureform einsetzbare Carbonsäuren sind beispielsweise Hexansäure (Capronsäure), Heptansäure (Önanthsäure), Octansäure (Caprylsäure), Nonansäure (Pelargonsäure), Decansäure (Caprinsäure), Undecansäure usw.. Bevorzugt ist im Rahmen der vorliegenden Verbindung der Einsatz von Fettsäuren wie Dodecansäure (Laurinsäure), Tetradecansäure (Myristinsäure), Hexadecansäure (Palmitinsäure), Octadecansäure (Stearinsäure), Eicosansäure (Arachinsäure), Docosansäure (Behensäure), Tetracosansäure (Lignocerinsäure), Hexacosansäure (Cerotinsäure), Triacotansäure (Melissinsäure) sowie der ungesättigten Spezies 9c-Hexadecensäure (Palmitoleinsäure), 6c-Octadecensäure (Petroselinsäure), 6t-Octadecensäure (Petroselaidinsäure), 9c-Octadecensäure (Ölsäure), 9t-Octadecensäure ((Elaidinsäure), 9c,12c-Octadecadiensäure (Linolsäure), 9t,12t-Octadecadiensäure (Linolaidinsäure) und 9c,12c,15c-Octadecatriensäure (Linolensäure). Aus Kostengründen ist es bevorzugt, nicht die reinen Spezies einzusetzen, sondern technische Gemische der einzelnen Säuren, wie sie aus der Fettspaltung zugänglich sind. Solche Gemische sind beispielsweise Kokosölfettsäure (ca: 6 Gew.-% C₈, 6 Gew.-% C₁₀, 48 Gew.-%C 12, 18 Gew.-%C₁₄, 10 Gew.-% C₁₆, 2 Gew.-% C₁₈, 8 Gew.-% C₁₈, 1 Gew.-% C_{18''}), Palmkernölfettsäure (ca. 4 Gew.-% C₈, 5 Gew.-% C₁₀, 50 Gew.-% C₁₂, 15 Gew.-% C₁₄, 7 Gew.-% C₁₆, 2 Gew.-% C₁₈, 15 Gew.-% C_{18'}, 1 Gew.-% C₁₈), Talgfettsäure (ca. 3 Gew.-% C₁₄, 26 Gew.-% C₁₆, 2 Gew.-% C_{16'}, 2 Gew.-% C₁₇, 17 Gew.-% C₁₈, 44 Gew.-% C_{18'}, 3 Gew.-% C_{18''}, 1 Gew.-% C_{18'''}), gehärtete Talgfettsäure (ca. 2 Gew.-% C₁₄, 28 Gew.-% C₁₆, 2 Gew.-% C₁₇, 63 Gew.-% C₁₈, 1 Gew.-% C_{18'}), technische Ölsäure (ca. 1 Gew.-% C₁₂, 3 Gew.-% C₁₄, 5 Gew.-% C₁₆, 6 Gew.-% C_{16'}, 1 Gew.-% C₁₇, 2 Gew.-% C₁₈, 70 Gew.-% C₁₈, 10 Gew.-% C_{18''}, 0,5 Gew.-% C_{18'''}), technische Palmitin/Stearinsäure (ca. 1 Gew.-% C₁₂, 2 Gew.-% C₁₄, 45 Gew.-% C₁₆, 2 Gew.-% C₁₇, 47 Gew.-% C₁₈, 1 Gew.-% C_{18'}) sowie Sojabohnenölfettsäure (ca. 2 Gew.-% C₁₄, 15 Gew.-% C₁₆, 5 Gew.-% C₁₈, 25 Gew.-% C_{18'}, 45 Gew.-% C_{18''}, 7 Gew.-% C_{18'''}).

Schwefelsäurehalbester längerkettiger Alkohole sind ebenfalls Aniontenside in ihrer Säureform und im Rahmen der vorliegenden Erfindung einsetzbar. Ihre Alkalimetall-, insbesondere Natriumsalze, die Fettalkoholsulfate, sind großtechnisch aus Fettalkoholen zugänglich, welche mit Schwefelsäure, Chlorsulfonsäure, Amidosulfonsäure oder Schwefeltrioxid zu den betreffenden Alkylschwefelsäuren umgesetzt und nachfolgend neutralisiert werden. Die Fettalkohole werden dabei aus den betreffenden Fettsäuren bzw. Fettsäuregemischen durch Hochdruckhydrierung der Fettsäuremethylester gewonnen. Der mengenmäßig bedeutendste industrielle Prozeß zur Herstellung von Fettalkylschwefelsäuren ist die Sulfierung der Alkohole mit SO₃/Luft-Gemischen in speziellen Kaskaden-, Fallfilm- oder Röhrenbündelreaktoren.

Eine weitere Klasse von Aniontensidsäuren, die erfindungsgemäß eingesetzt werden kann, sind die Alkyletherschwefelsäuren, deren Salze, die Alkylethersulfate, sich im Vergleich zu den Alkylsulfaten durch eine höhere Wasserlöslichkeit und geringere Empfindlichkeit gegen Wasserhärte (Löslichkeit der Ca-Salze) auszeichnen. Alkyletherschwefelsäuren werden wie die Alkylschwefelsäuren aus Fettalkoholen synthetisiert, welche mit Ethylenoxid zu den betreffenden Fettalkoholethoxylaten umgesetzt werden. Anstelle von Ethylenoxid kann auch Propylenoxid eingesetzt werden. Die nachfolgende Sulfonierung mit gasförmigem Schwefeltrioxid in Kurzzeit-Sulfierreaktoren liefert Ausbeuten über 98% an den betreffenden Alkyletherschwefelsäuren.

Auch Alkansulfonsäuren und Olefinsulfonsäuren sind im Rahmen der vorliegenden Erfindung als Aniontenside in Säureform einsetzbar. Alkansulfonsäuren können die Sulfonsäuregruppe terminal gebunden (primäre Alkansulfonsäuren) oder entlang der C-Kette enthalten (sekundäre Alkansulfonsäuren), wobei lediglich die sekundären Alkansulfonsäuren kommerzielle Bedeutung besitzen. Diese werden durch Sulfochlorierung oder Sulfoxidation linearer Kohlenwasserstoffe hergestellt. Bei der Sulfochlorierung nach Reed werden n-Paraffine mit Schwefeldioxid und Chlor unter Bestrahlung mit UV-Licht zu den entsprechenden Sulfochloriden umgesetzt, die bei Hydrolyse mit Alkalien direkt die Alkansulfonate, bei Umsetzung mit Wasser die Alkansulfonsäuren, liefern. Da bei der Sulfochlorierung Di- und Polysulfochloride sowie Chlorkohlenwasserstoffe als Nebenprodukte der radikalischen Reaktion auftreten können, wird die Reaktion üblicherweise nur bis zu Umsetzungsgraden von 30% durchgeführt und danach abgebrochen.

Ein anderer Prozeß zur Herstellung von Alkansulfonsäuren ist die Sulfoxidation, bei der n-Paraffine unter Bestrahlung mit UV-Licht mit Schwefeldioxid und Sauerstoff umgesetzt werden. Bei dieser Radikalreaktion entstehen sukzessive Alkylsulfonylradikale, die mit Sauerstoff zu den Alkylpersulfonylradikalen weiter reagieren. Die Reaktion mit unumgesetztem Paraffin liefert ein Alkylradikal und die Alkylpersulfonsäure, welche in ein Alkylperoxysulfonylradikal und ein Hydroxylradikal zerfällt. Die Reaktion der beiden Radikale mit unumgesetztem Paraffin liefert die Alkylsulfonsäuren bzw. Wasser, welches mit Alkylpersulfonsäure und Schwefeldioxid zu Schwefelsäure reagiert. Um die Ausbeute an den beiden Endprodukten Alkylsulfonsäure und Schwefelsäure möglichst hoch zu halten und Nebenreaktionen zu unterdrücken, wird diese Reaktion üblicherweise nur bis zu Umsetzungsgraden von 1% durchgeführt und danach abgebrochen.

Olefinsulfonate werden technisch durch Reaktion von α-Olefinen mit Schwefeltrioxid hergestellt. Hierbei bilden sich intermediär Zwitterionen, welche sich zu sogenannten Sultonen cyclisieren. Unter geeigneten Bedingungen (alkalische oder saure Hydrolyse) reagieren diese Sultone zu Hydroxylaikansuifonsäuren bzw. Alkensulfonsäuren, welche beide ebenfalls als Aniontensidsäuren eingesetzt werden können.

Alkylbenzolsulfonate als leistungsstarke anionische Tenside sind seit den dreißiger Jahren des letzten Jahrhunderts bekannt. Damals wurden durch Monochlorierung von Kogasin-Fraktionen und anschließende Friedel-Crafts-Alkylierung Alkylbenzole hergestellt, die mit Oleum sulfoniert und mit Natronlauge neutralisiert wurden. Anfang der fünfziger Jahre wurde zur Herstellung von Alkylbenzolsulfonaten Propylen zu verzweigtem α-Dodecylen tetramerisiert und das Produkt über eine Friedel-Crafts-Reaktion unter Verwendung von Aluminiumtrichlorid oder Fluorwasserstoff zum Tetrapropylenbenzol umgesetzt, das nachfolgend sulfoniert und neutralisiert wurde. Diese ökonomische Möglichkeit der Herstellung von Tetrapropylenbenzolsulfonaten (TPS) führte zum Durchbruch dieser Tensidklasse, die nachfolgend die Seifen als Haupttensid in Wasch- und Reinigungsmitteln verdrängte.

Aufgrund der mangelnden biologischen Abbaubarkeit von TPS bestand die Notwendigkeit, neue Alkylbenzolsulfonate darzustellen, die sich durch ein verbessertes ökologische Verhalten auszeichnen. Diese Erfordernisse werden von linearen Alkylbenzolsulfonaten erfüllt, welche heute die fast ausschließlich hergestellten Alkylbenzolsulfonate sind und mit dem Kurzzeichen ABS bzw. LAS belegt werden.

Lineare Alkylbenzolsulfonate werden aus linearen Alkylbenzolen hergestellt, welche wiederum aus linearen Olefinen zugänglich sind. Hierzu werden großtechnisch Petroleumfraktionen mit Molekularsieben in die n-Paraffine der gewünschten Reinheit aufgetrennt und zu den n-Olefinen dehydriert, wobei sowohl α- als auch i-Olefine resultieren. Die entstandenen Olefine werden dann in Gegenwart saurer Katalysatoren mit Benzol zu den Alkylbenzolen umgesetzt, wobei die Wahl des Friedel-Crafts-Katalysators einen Einfluß auf die lsomerenverteilung der entstehenden linearen Alkylbenzole hat: Bei Verwendung von Aluminiumtrichlorid liegt der Gehalt der 2-Phenyl-Isomere in der Mischung mit den 3-, 4-, 5- und anderen isomeren bei ca. 30 Gew.%, wird hingegen Fluorwasserstoff als Katalysator eingesetzt, läßt sich der Gehalt an 2-Phenyl-Isomer auf ca. 20 Gew.-% senken. Die Sulfonierung der linearen Alkylbenzole schließlich gelingt heute großtechnisch mit Oleum, Schwefelsäure oder gasförmigem Schwefeltrioxid, wobei letzteres die weitaus größte Bedeutung hat. Zur Sulfonierung werden spezielle Film- oder Rohrbündelreaktoren eingesetzt, die als Produkt eine 97 Gew.-%ige Alkylbenzolsulfonsäure (ABSS) liefern, die im Rahmen der vorliegenden Erfindung als Aniontensidsäure einsetzbar ist.

Durch Wahl des Neutralisationsmittels lassen sich aus den ABSS die unterschiedlichsten Salze, d.h. Alkylbenzolsulfonate, gewinnen. Aus Gründen der Ökonomie ist es hierbei bevorzugt, die Alkalimetallsalze und unter diesen bevorzugt die Natriumsalze der ABSS herzustellen und einzusetzen. Diese lassen sich durch die allgemeine Formel IX beschreiben: 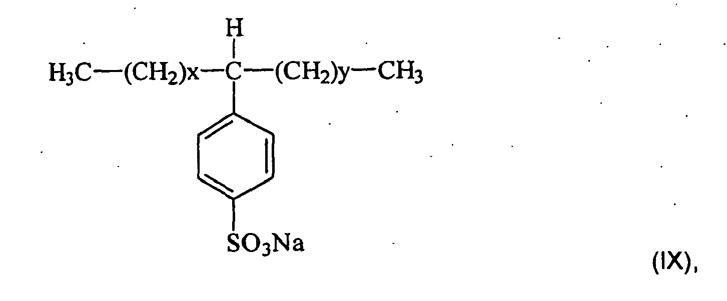 in der die Summe aus x und y üblicherweise zwischen 5 und 13 liegt. Erfindungsgemäß bevorzugt als Aniontensid in Säureform sind C₈₋₁₆-, vorzugsweise C₉₋₁₃-Alkylbenzolsulfonsäuren. Es ist im Rahmen der vorliegenden Erfindung weiterhin bevorzugt, C₈₋₁₆-, vorzugsweise C₉₋₁₃-Alkybenzolsulfonsäuren einzusetzen, die sich von Alkylbenzolen ableiten, welche einen Tetralingehalt unter 5 Gew.-%, bezogen auf das Alkylbenzol, aufweisen. Weiterhin bevorzugt ist es, Alkylbenzolsulfonsäuren zu verwenden, deren Alkylbenzole nach dem HF-Verfahren hergestellt wurden, so daß die eingesetzten C₈₋₁₆-, vorzugsweise C₉₋₁₃-Alkybenzolsulfonsäuren einen Gehalt an 2-Phenyl-Isomer unter 22 Gew.-%, bezogen auf die Alkylbenzolsulfonsäure, aufweisen.

Die vorstehend genannten Aniontenside in ihrer Säureform können alleine oder in Mischung miteinander eingesetzt werden. Es ist aber auch möglich und bevorzugt, daß dem Aniontensid in Säureform vor der Zugabe auf das/die Trägermateriat(ien) weitere, vorzugsweise saure, Inhaltsstoffe von Wasch- und Reinigungsmitteln in Mengen von 0,1 bis 40 Gew.-%, vorzugsweise von 1 bis 15 Gew.-% und insbesondere von 2 bis 10 Gew.-%, jeweils bezogen auf das Gewicht der umzusetzenden Mischung, zugemischt werden.

Selbstverständlich ist es auch möglich, die Aniontenside teil- oder vollneutralisiert einzusetzen: Diese Salze können dann als Lösung, Suspension oder Emulsion in der Granulierflüssigkeit vorliegen, aber auch als Feststoff Bestandteil des Feststoffbetts sein. Als Kationen für solche Aniontenside bieten sich neben den Alkalimetallen (hier insbesondere nach Anspruch- und K-Salze) Ammonium- sowie Mono-, Di- oder Triethanolalkonium-lonen an. Anstelle von Mono-, Di- oder Triethanolamin können auch die analogen Vertreter des Mono-, Di- oder Trimethanolamins bzw. solche der Alkanolamine höherer Alkohole quaterniert und als Kation zugegen sein.

Auch Kationtenside lassen sich mit Vorteil als Aktivsubstanz einsetzen. Das Kationtensid kann dabei in seiner Lieferform direkt in den Mischer gegeben werden, oder in Form einer flüssigen bis pastösen Kationtensid-Zubereitungsform auf den festen Träger aufgedüst werden. Solche Kationtensid-Zubereitungsformen lassen sich beispielsweise durch Mischen handelsüblicher Kationtenside mit Hilfsstoffen wie nichtionischen Tensiden, Polyethylenglycolen oder Polyolen herstellen. Auch niedere Alkohole wie Ethanol und Isopropanol können eingesetzt werden, wobei die Menge an solchen niederen Alkoholen in der flüssigen Kationtensid-Zubereitungsform aus den obengenannten Gründen unter 10 Gew.-% liegen sollte.

Als Kationtenside kommen für die erfindungsgemäßen Mittel alle üblichen Stoffe in Betracht, wobei Kationtenside mit textilweichmachender Wirkung deutlich bevorzugt sind.

Die erfindungsgemäßen Mittel können als kationische Aktivsubstanzen mit textilweichmachender Wirkung ein oder mehrere kationische, textilweichmachende Mittel der Formeln X, XI oder XII enthalten: 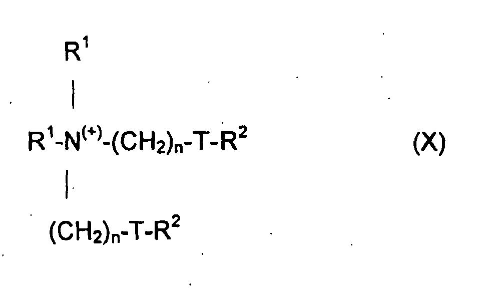 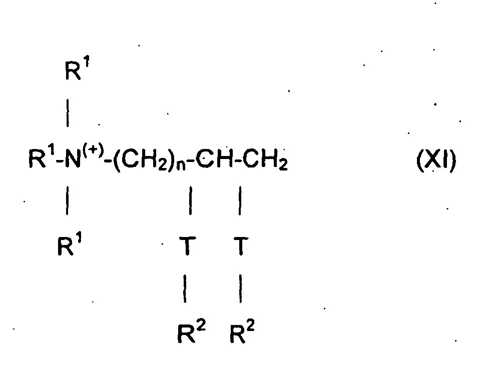 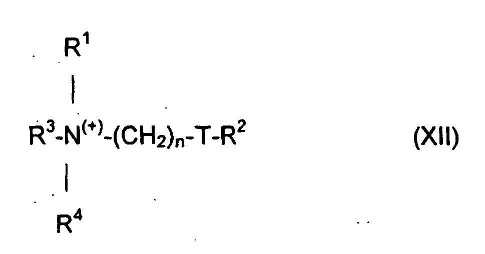 worin jede Gruppe R¹ unabhängig voneinander ausgewählt ist aus C₁₋₆-Alkyl-, -Alkenyl- oder -Hydroxyalkylgruppen; jede Gruppe R² unabhängig voneinander ausgewählt ist aus C₈₋₂₈-Alkyl- oder -Alkenylgruppen; R³ = R¹ oder (CH₂)ₙ-T-R²; R⁴ = R¹ oder R² oder (CH₂)ₙ-T-R²; T = -CH₂-, -O-CO- oder -CO-O- und n eine ganze Zahl von 0 bis 5 ist.

In bevorzugten Ausführungsformen der vorliegenden Erfindung enthalten die Mittel zusätzlich Niotensid(e) als Aktivsubstanz.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Außerdem können als weitere nichtionische Tenside auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel XIII,

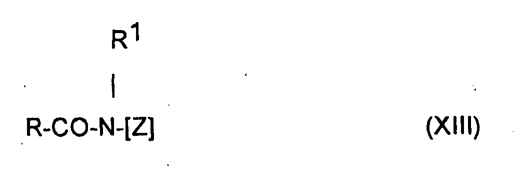

in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel XIV,

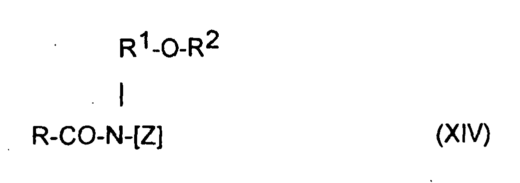

in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierten. Zuckers erhalten, beispielsweise Glucose, Fructose, Maltose, Lactose, Galactose, Mannose oder Xylose. Die N-Alkoxy- oder N-Aryloxy-substituierten Verbindungen können dann durch Umsetzung mit Fettsäuremethylestern in Gegenwart eines Alkoxids als Katalysator in die gewünschten Polyhydroxyfettsäureamide überführt werden.

Es ist für viele Anwendungen besonders bevorzugt, wenn das Verhältnis von Aniontensid(en) zu Niotensid(en) zwischen 10:1 und 1:10, vorzugsweise zwischen 7,5:1 und 1:5 und insbesondere zwischen 5:1 und 1:2 beträgt. Bevorzugt sind dabei erfindungsgemäße Behälter, die Tensid(e), vorzugsweise anionische(s) und/oder nichtionische(s) Tensid(e), in Mengen von 5 bis 80 Gew.-%, vorzugsweise von 7,5 bis 70 Gew.-%, besonders bevorzugt von 10 bis 60 Gew.-% uns insbesondere von 12,5 bis 50 Gew.-%, jeweils bezogen auf das Gewicht der umschlossenen Feststoffe, enthalten.

Wie bereits erwähnt, beschränkt sich der Einsatz von Tensiden bei Reinigungsmitteln für das maschinelle Geschirrspülen vorzugsweise auf den Einsatz nichtionischer Tenside in geringen Mengen. Erfindungsgemäße Mittel für das maschinelle Geschirrspülen enthalten daher vorzugsweise nur bestimmte nichtionische Tenside, die nachstehend beschrieben sind. Als Tenside werden in maschinellen Geschirrspülmitteln üblicherweise lediglich schwachschäumende nichtionische Tenside eingesetzt. Vertreter aus den Gruppen der anionischen, kationischen oder amphoteren Tenside haben dagegen eine geringere Bedeutung. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohof mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Insbesondere bei Reinigungsmitteln für das maschinelle Geschirrspülen ist es bevorzugt, daß diese ein nichtionisches Tensid enthalten, das einen Schmelzpunkt oberhalb Raumtemperatur aufweist, bevorzugt ein nichtionisches Tensid mit einem Schmelzpunkt oberhalb von 20°C. Bevorzugt einzusetzende nichtionische Tenside weisen Schmelzpunkte oberhalb von 25°C auf, besonders bevorzugt einzusetzende nichtionische Tenside haben Schmelzpunkte zwischen 25 und 60°C, insbesondere zwischen 26,6 und 43,3°C.

Geeignete nichtionische Tenside, die Schmelz- bzw. Erweichungspunkte im genannten Temperaturbereich aufweisen, sind beispielsweise schwachschäumende nichtionische Tenside, die bei Raumtemperatur fest oder hochviskos sein können. Werden bei Raumtemperatur hochviskose Niotenside eingesetzt, so ist bevorzugt, daß diese eine Viskosität oberhalb von 20 Pas, vorzugsweise oberhalb von 35 Pas und insbesondere oberhalb 40 Pas aufweisen. Auch Niotenside, die bei Raumtemperatur wachsartige Konsistenz besitzen, sind bevorzugt.

Bevorzugt als bei Raumtemperatur feste einzusetzende Niotenside stammen aus den Gruppen der alkoxylierten Niotenside, insbesondere der ethoxylierten primären Alkohole und Mischungen dieser Tenside mit strukturell komplizierter aufgebauten Tensiden wie Polyoxypropylen/Polyoxyethylen/Polyoxypropylen (PO/EO/PO)-Tenside. Solche (PO/EO/PO)-Niotenside zeichnen sich darüber hinaus durch gute Schaumkontrolle aus.

In einer bevorzugten Ausführungsform der vorliegenden Erfindung ist das nichtionische Tensid mit einem Schmelzpunkt oberhalb Raumtemperatur ein ethoxyliertes Niotensid, das aus der Reaktion von einem Monohydroxyalkanol oder Alkylphenol mit 6 bis 20 C-Atomen mit vorzugsweise mindestens 12 Mol, besonders bevorzugt mindestens 15 Mol, insbesondere mindestens 20 Mol Ethylenoxid pro Mol Alkohol bzw. Alkylphenol hervorgegangen ist.

Ein besonders bevorzugtes bei Raumtemperatur festes, einzusetzendes Niotensid wird aus einem geradkettigen Fettalkohol mit 16 bis 20 Kohlenstoffatomen (C₁₆₋₂₀-Alkohol), vorzugsweise einem C₁₈-Alkohol und mindestens 12. Mol, vorzugsweise mindestens 15 Mol und insbesondere mindestens 20 Mol Ethylenoxid gewonnen. Hierunter sind die sogenannten "narrow range ethoxylates" (siehe oben) besonders bevorzugt.

Das bei Raumtemperatur feste Niotensid besitzt vorzugsweise zusätzlich Propylenoxideinheiten im Molekül. Vorzugsweise machen solche PO-Einheiten bis zu 25 Gew.-%, besonders bevorzugt bis zu 20 Gew.-% und insbesondere bis zu 15 Gew.-% der gesamten Molmasse des nichtionischen Tensids aus. Besonders bevorzugte nichtionische Tenside sind ethoxylierte Monohydroxyalkanole oder Alkylphenole, die zusätzlich Polyoxyethylen-Polyoxypropylen Blockcopolymereinheiten aufweisen. Der Alkohol- bzw. Alkylphenolteil solcher Niotensidmoleküle macht dabei vorzugsweise mehr als 30 Gew.-%, besonders bevorzugt mehr als 50 Gew.-% und insbesondere mehr als 70 Gew.-% der gesamten Molmasse solcher Niotenside aus.

Weitere besonders bevorzugt einzusetzende Niotenside mit Schmelzpunkten oberhalb Raumtemperatur enthalten 40 bis 70% eines Polyoxypropylen/Polyoxyethylen/Polyoxypropylen-Blockpolymerblends, der 75 Gew.-% eines umgekehrten Block-Copolymers von Polyoxyethylen und Polyoxypropylen mit 17 Mol Ethylenoxid und 44 Mol Propylenoxid und 25 Gew.-% eines Block-Copolymers von Polyoxyethylen und Polyoxypropylen, initiiert mit Trimethylolpropan und enthaltend 24 Mol Ethylenoxid und 99 Mol Propylenoxid pro Mol Trimethylolpropan.

Nichtionische Tenside, die mit besonderem Vorzug eingesetzt werden können, sind beispielsweise unter dem Namen Poly Tergent^{®} SLF-18 von der Firma Olin Chemicals erhältlich.

Ein weiter bevorzugtes Tensid, läßt sich durch die Formel

R¹O[CH₂CH(CH₃)O]ₓ[CH₂CH₂O]_{y}[CH₂CH(OH)R²]

beschreiben, in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 und y für einen Wert von mindestens 15 steht.

Weitere bevorzugt einsetzbare Niotenside sind die endgruppenverschlossenen Poly(oxyalkylierten) Niotenside der Formel

R¹O[CH₂CH(R³)O]ₓ[CH₂]ₖCH(OH)[CH₂]ⱼOR²

in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der obenstehenden Formel unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der obenstehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Insbesondere bevorzugte endgruppenverschlossenen Poly(oxyalkylierte) Alkohole der obenstehenden Formel weisen Werte von k = 1 und j = 1 auf, so daß sich die vorstehende Formel zu

R¹O[CH₂CH(R³)O]_{X}CH₂CH(OH)CH₂OR²

vereinfacht. In der letztgenannten Formel-sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Erfindungsgemäße Mittel können zur Steigerung der Wasch-, beziehungsweise Reinigungsleistung Enzyme enthalten, wobei prinzipiell alle im Stand der Technik für diese Zwecke etablierten Enzyme einsetzbar sind. Hierzu gehören insbesondere Proteasen, Amylasen, Lipasen, Hemicellulasen, Cellulasen oder Oxidoreduktasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Erfindungsgemäße Mittel enthalten Enzyme vorzugsweise in Gesamtmengen von 1 x 10⁻⁶ bis 5 Gewichts-Prozent bezogen auf aktives Protein. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden.

Unter den Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von der Firma Novozymes A/S, Bagsvaerd, Dänemark, erhältlich. Die Subtilisine 147 und 309 werden unter den Handelsnamen Esperase^{®}, beziehungsweise Savinase^{®} von der Firma Novozymes vertrieben. Von der Protease aus *Bacillus lentus* DSM 5483 leiten sich die unter der Bezeichnung BLAP^{®} geführten Varianten ab.

Weitere brauchbare Proteasen sind beispielsweise die unter den Handelsnamen Durazym^{®}, Relase^{®}, Everlase^{®}, Nafizym, Natälase^{®}, Kannase^{®} und Ovozymes^{®} von der Firma Novozymes, die unter den Handelsnamen, Purafect^{®}, Purafect^{®}OxP und Properase^{®} von der Firma Genencor, das unter dem Handelsnamen Protosol^{®} von der Firma Advanced Biochemicals Ltd., Thane, Indien, das unter dem Handelsnamen Wuxi^{®} von der Firma Wuxi Snyder Bioproducts Ltd., China, die unter den Handelsnamen Proleather^{®} und Protease P^{®} von der Firma Arnano Pharmaceuticals Ltd., Nagoya, Japan, und das unter der Bezeichnung Proteinase K-16 von der Firma Kao Corp., Tokyo, Japan, erhältlichen Enzyme.

Beispiele für erfindungsgemäß einsetzbare Amylasen sind die α-Amylasen aus *Bacillus licheniformis,* aus *B. amyloliquefaciens* oder aus *B. stearothermophilus* sowie deren für den Einsatz in Wasch- und Reinigungsmitteln verbesserte Weiterentwicklungen. Das Enzym aus *B. licheniformis* ist von der Firma Novozymes unter dem Namen Termamyl^{®} und von der Firma Genencor unter dem Namen Purastar^{®}ST erhältlich. Weiterentwicklungsprodukte dieser α-Amylase sind von der Firma Novozymes unter den Handelsnamen Duraniyl^{®} und Termamyl^{®}ultra, von der Firma Genencor unter dem Namen Purastar^{®}OxAm und von der Firma Daiwa Seiko Inc., Tokyo, Japan, als Keistase^{®} erhältlich. Die α-Amylase von *B. amyloliquefaciens* wird von der Firma Novozymes unter dem Namen BAN^{®} vertrieben, und abgeleitete Varianten von der α-Amylase aus *B. stearothermophilus* unter den Namen BSG^{®} und Novamyl^{®}, ebenfalls von der Firma Novozymes.

Des weiteren sind für diesen Zweck die α-Amylase aus *Bacillus sp.* A 7-7 (DSM 12368) und die Cyctodextrin-Glucanotransferase (CGTase) aus *B. agaradherens* (DSM 9948) hervorzuheben; ebenso sind Fusionsprodukte der genannten Moleküle einsetzbar.

Darüber hinaus sind die unter den Handelsnamen Fungamyl^{®} von der Firma Novozymes erhältlichen Weiterentwicklungen der α-Amylase aus *Aspergillus niger* und A. oryzae geeignet. Ein weiteres Handelsprodukt ist beispielsweise die Amylase-LT^{®}.

Erfindungsgemäße Mittel können Lipasen oder Cutinasen insbesondere wegen ihrer Triglycerid-spaltenden Aktivitäten enthalten, aber auch, um aus geeigneten Vorstufen *in situ* Persäuren zu erzeugen. Hierzu gehören beispielsweise die ursprünglich aus *Humicola lanuginosa* (*Thermomyces lanuginosus*) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase^{®}, Lipolase^{®}Ultra, LipoPrime^{®}, Lipozyme^{®} und Lipex^{®} vertrieben. Desweiteren sind beispielsweise die Cutinasen einsetzbar, die ursprünglich aus *Fusarium solani pisi* und *Humicola insolens* isoliert worden sind. Ebenso brauchbare Lipasen sind von der Firma Amano unter den Bezeichnungen Lipase CE^{®}, Lipase P^{®}, Lipase B^{®}, beziehungsweise Lipase CES^{®}, Lipase AKG^{®}, Bacillis sp. Lipase^{®}, Lipase AP^{®}, Lipase M-AP^{®}und Lipase AML^{®} erhältlich. Von der Firma Genencor sind beispielsweise die Lipasen beziehungsweise Cutinasen einsetzbar, deren Ausgangsenzyme ursprünglich aus *Pseudomonas mendocina* und *Fusarium solanii* isoliert worden sind. Als weitere wichtige Handelsprodukte sind die ursprünglich von der Firma Gist-Brocades vertriebenen Präparationen M1 Lipase^{®} und Lipomax^{®} und die von der Firma Meito Sangyo KK, Japan, unter den Namen Lipase MY-30^{®}, Lipase OF^{®} und Lipase PL^{®} vertriebenen Enzyme zu erwähnen, ferner das Produkt Lumafast^{®} von der Firma Genencor.

Erfindungsgemäße Mittel können, insbesondere wenn sie für die Behandlung von Textilien gedacht sind, Cellulasen enthalten, je nach Zweck als reine Enzyme, als Enzympräparationen oder in Form von Mischungen, in denen sich die einzelnen Komponenten vorteilhafterweise hinsichtlich ihrer verschiedenen Leistungsaspekte ergänzen. Zu diesen Leistungsaspekten zählen insbesondere Beiträge zur Primärwaschleistung, zur Sekundärwaschleistung des Mittels (Antiredepositionswirkung oder Vergrauungsinhibition) und Avivage (Gewebewirkung), bis hin zum Ausüben eines "stone washed"-Effekts.

Eine brauchbare pilzliche, Endoglucanase(EG)-reiche Cellulase-Präparation, beziehungsweise deren Weiterentwicklungen werden von der Firma Novozymes unter dem Handelsnamen Celluzyme^{®} angeboten. Die ebenfalls von der Firma Novozymes erhältlichen Produkte Endolase^{®} und Carezyme^{®} basieren auf der 50 kD-EG, beziehungsweise der 43 kD-EG aus *H. insolens* DSM 1800. Weitere mögliche Handelsprodukte dieser Firma sind Cellusoft^{®} und Renozyme^{®}. Ebenso ist die 20 kD-EG Cellulase aus *Melanocarpus,* die von der Firma AB Enzymes, Finnland, unter den Handelsnamen Ecostone^{®} und Biotouch^{®} erhältlich ist, einsetzbar. Weitere Handelprodukte der Firma AB Enzymes sind Econase^{®} und Ecopulp^{®}. Eine weitere geeignete Cellulase aus *Bacillus sp.* CBS 670.93 ist von der Firma Genencor unter dem Handelsnamen Puradax^{®} erhältlich. Weitere Handelsprodukte der Firma Genencor sind "Genencor detergent cellulase L" und IndiAge^{®}Neutra.

Erfindungsgemäße Mittel können weitere Enzyme enthalten, die unter dem Begriff Hemicellulasen zusammengefaßt werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen. Geeignete Mannanasen sind beispielsweise unter den Namen Gamanase^{®} und Pektinex AR^{®} von der Firma Novozymes, unter dem Namen Rohapec^{®} B1 L von der Firma AB Enzymes und unter dem Namen Pyrolase^{®} von der Firma Diversa Corp., San Diego, CA, USA erhältlich. Die aus *B. subtilis* gewonnene β-Glucanase ist unter dem Namen Cereflo^{®} von der Firma Novozymes erhältlich.

Zur Erhöhung der bleichenden Wirkung können erfindungsgemäße Wasch- oder Reinigungsmittel Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) enthalten. Als geeignete-Handelsprodukte sind Denilite^{®} 1 und 2 der Firma Novozymes zu nennen. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die in erfindungsgemäßen Mitteln eingesetzten Enzyme stammen entweder ursprünglich aus Mikroorganismen, etwa der Gattungen *Bacillus, Streptomyces, Humicola,* oder *Pseudomonas,* und/oder werden nach an sich bekannten biotechnologischen Verfahren durch geeignete Mikroorganismen produziert, etwa durch transgene Expressionswirte der Gattungen *Bacillus* oder filamentöse Fungi.

Die Aufreinigung der betreffenden Enzyme erfolgt günstigerweise über an sich etablierte Verfahren, beispielsweise über Ausfällung, Sedimentation, Konzentrierung, Filtration der flüssigen Phasen, Mikrofiltration, Ultrafiltration, Einwirken von Chemikalien, Desodorierung oder geeignete Kombinationen dieser Schritte.

Erfindungsgemäßen Mitteln können die Enzyme in jeder nach dem Stand der Technik etablierten Form zugesetzt werden. Hierzu gehören beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem, vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalienundurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so daß ein einzelnes Granulat mehrere Enzymaktivitäten aufweist.

Ein in einem erfindungsgemäßen Mittel enthaltenes Protein und/oder Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Erfindungsgemäße Mittel können zu diesem Zweck Stabilisatoren enthalten; die Bereitstellung derartiger Mittel stellt eine bevorzugte Ausführungsform der vorliegenden Erfindung dar.

Eine Gruppe von Stabilisatoren sind reversible Proteaseinhibitoren. Häufig werden Benzamidin-Hydrochlorid, Borax, Borsäuren, Boronsäuren oder deren Salze oder Ester verwendet, darunter vor allem Derivate mit aromatischen Gruppen, etwa ortho-,meta- oder para-substituierte Phenylboronsäuren, beziehungsweise deren Salze oder Ester. Weiterhin sind Peptid aldehyde, das heißt Oligopeptide mit reduziertem C-Terminus geeignet. Als peptidische Proteaseinhibitoren sind unter anderem Ovomucoid und Leupeptin zu erwähnen; eine zusätzliche Option ist die Bildung von Fusionsproteinen aus Proteasen und Peptid-Inhibitoren.

Weitere Enzymstabilisatoren sind Aminoalkohole wie Mono-, Di-, Triethanol- und - Propanolamin und deren Mischungen, aliphatische Carbonsäuren bis zu C₁₂, wie Bernsteinsäure, andere Dicarbonsäuren oder Salze der genannten Säuren. Auch endgruppenverschlossene Fettsäureamidalkoxylate sind als Stabilisatoren einsetzbar.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Weiterhin schützt auch Di-Glycerinphosphat gegen Denaturierung durch physikalische Einflüsse. Ebenso werden Calciumsalze verwendet, wie beispielsweise Calciumacetat oder Calcium-Formiat sowie Magnesiumsalze.

Polyamid-Oligomere oder polymere Verbindungen wie Lignin, wasserlösliche Vinyl-Copolymere oder, wie Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind die linearen C₈-C₁₈ Polyoxyalkylene. Alkylpolyglycoside können gemäß den ebenfalls die enzymatischen Komponenten des erfindungsgemäßen Mittels stabilisieren und sogar in ihrer Leistung steigern. Vernetzte N-haltige Verbindungen erfüllen eine Doppelfunktion als Soil-release-Agentien und als Enzym-Stabilisatoren.

Reduktionsmittel und Antioxidantien wie Natrium-Sulfit oder reduzierende Zucker erhöhen die Stabilität der Enzyme gegenüber oxidativern Zerfall.

Bevorzugt werden Kombinationen von Stabilisatoren verwendet, beispielsweise aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren kann durch die Kombination mit Borsäure und/oder Borsäurederivaten und Polyolen gesteigert und gemäß durch die zusätzliche Verwendung von zweiwertigen Kationen, wie zum Beispiel Calcium-Ionen weiter verstärkt werden.

Besonders bevorzugt ist im Rahmen der vorliegenden Erfindung der Einsatz flüssiger Enzymformulierungen. Hier sind erfindungsgemäße Mittel bevorzugt, die zusätzlich Enzyme und/oder Enzymzubereitungen, vorzugsweise feste und/oder flüssige Protease-Zubereitungen und/oder Amylase-Zubereitungen, in Mengen von 1 bis 5 Gew.-%, vorzugsweise von 1,5 bis 4,5 und insbesondere von 2 bis 4 Gew.-%, jeweils bezogen auf das gesamte Mittel, enthalten.

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den erfindungsgemäßen Granulaten bevorzugt.

Um den pH-Wert von Lösungen der erfindungsgemäßen Wasch- oder Reinigungsmittel in den gewünschten Bereich zu bringen, kann der Einsatz von pH-Stellmitteln angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 1 Gew.-% der Gesamtformulierung nicht.

Als Hydrotrope oder Lösungsvermittler werden Substanzen bezeichnet, die durch ihre Gegenwart andere, in einem bestimmten Lösungsmittel praktisch unlösliche Verbindungen in diesem Lösungsmittel löslich oder emulgierbar machen (Solubilisation). Es gibt Lösungsvermittler, die mit der schwerlöslichen Substanz eine Molekülverbindung eingehen und solche, die durch Micell-Bildung wirken. Man kann auch sagen, daß erst Lösungsvermittler einem sogenannten latenten Lösemittel sein Lösungsvermögen verleihen. Bei Wasser als (latentem) Lösungsmittel spricht man statt von Lösungsvermittler meist von Hydrotropika, in bestimmten Fällen besser von Emulgatoren.

Als Schaumirihibitoren, die in den erfindungsgemäßen Mitteln eingesetzt werden können, kommen u.a. Seifen, Öle, Fette, Paraffine oder Silikonöle in Betracht, die gegebenenfalls auf Trägermaterialien aufgebracht sein können. Als Trägermaterialien eignen-sich beispielsweise anorganische Salze wie Carbonate oder Sulfate, Cellulosederivate oder Silikate sowie Mischungen der vorgenannten Materialien. Im Rahmen der vorliegenden Anmeldung bevorzugte Mittel enthalten Paraffine, vorzugsweise unverzweigte Paraffine (n-Paraffine) und/oder Silikone, vorzugsweise linear-polymere Silikone, welche nach dem Schema (R₂SiO)x aufgebaut sind und auch als Silikonöle bezeichnet werden. Diese Silikonöle stellen gewöhnlich klare, farblose, neutrale, geruchsfreie, hydrophobe Flüssigkeiten dar mit einem Molekulargewicht zwischen 1000-150 000, und Viskositäten zwischen 10 und 1 000 000 mPa · s.

Geeignete Antiredepositionsmittel, die auch als soil repellents bezeichnet werden, sind beispielsweise nichtionische Celluloseether wie Methylcellulose und Methylhydroxypropylcellulose mit einem Anteil an Methoxygruppen von 15 bis 30 Gew.-% und an Hydroxypropylgruppen von 1 bis 15 Gew.-%, jeweils bezogen auf den nichtionischen Celluloseether sowie die aus dem Stand der Technik bekannten Polymere der Phthalsäure und/oder Terephthalsäure bzw. von deren Derivaten, insbesondere Polymere aus Ethylenterephthalaten und/oder Polyethylenglycolterephthalaten oder anionisch und/oder nichtionisch modifizierten Derivaten von diesen. Insbesondere bevorzugt von diesen sind die sulfonierten Derivate der Phthalsäure- und Terephthalsäure-Polymere.

Optische Aufheller (sogenannte "Weißtöner") können den erfindungsgemäßen Mitteln zugesetzt werden, um Vergrauungen und Vergilbungen der behandelten Textilien zu beseitigen. Diese Stoffe ziehen auf die Faser auf und bewirken eine Aufhellung und vorgetäuschte Bleichwirkung, indem sie unsichtbare Ultraviolettstrahlung in sichtbares längerwelliges Licht umwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiß ergibt. Geeignete Verbindungen stammen beispielsweise aus den Substanzklassen der 4,4'-Diamino-2,2'-stilbendisulfonsäuren (Flavonsäuren), 4,4'-Distyryl-biphenylen, Methylumbelliferone, Cumarine, Dihydrochinolinone, 1,3-Diarylpyrazoline, Naphthalsäureimide, Benzoxazol-, Benzisoxazol- und Benzimidazol-Systeme sowie der durch Heterocyclen substituierten Pyrenderivate.

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise die wasserlöslichen Salze polymerer Carbonsäuren, Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw. Auch Polyvinylpyrrolidon ist brauchbar. Als Vergrauungsinhibitoren einsetzbar sind weiterhin Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxy-methylcellulose und deren Gemische.

Da textile Flächengebilde, insbesondere aus Reyon, Zellwolle, Baumwolle und deren Mischungen, zum Knittern eigen können, weil die Einzelfasern gegen Durchbiegen, Knicken. Pressen und Quetschen quer zur Faserrichtung empfindlich sind, können die erfindungsgemäßen Mittel synthetische Knitterschutzmittel enthalten. Hierzu zählen beispielsweise synthetische Produkte auf der Basis von Fettsäuren, Fettsäureestern. Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester. Eine im besonderen Maße zur Textilausrüstung und Pflege geeignete Substanz ist das Baumwollsamenöl, welches beispielsweise durch Auspressen der braunen gereinigten Baumwollsamen und Raffination mit etwa 10%igem Natriumhydroxid oder durch Extraktion mit Hexan bei 60-70°C hergestellt werden kann. Derartige Baumwollöle enthalten 40 bis 55 Gew.-% Linolsäure, 16 bis 26 Gew.-% Ölsäure und 20 bis 26 Gew.-% Palmitinsäure. Weitere zur Faserglättung und Faserpflege besonders bevorzugte Mittel sind die Glyceride, insbesondere die Monoglyceride von Fettsäuren wie beispielsweise Glycerinmonooleat oder Glycerinmonostearat.

Zur Bekämpfung von Mikroorganismen können die erfindungsgemäßen Mittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarlylsulfonate, Halogenphenole und Phenolmercuriacetat, wobei bei den erfindungsgemäßen Mitteln auch gänzlich auf diese Verbindungen verzichtet werden kann.

Um unerwünschte, durch Sauerstoffeinwirkung und andere oxidative Prozesse verursachte Veränderungen an den Wasch- und Reinigungsmitteln und/oder den behandelten Textilien zu verhindern, können die erfindungsgemäßen Mittel Antioxidantien enthalten. Zu dieser Verbindungsklasse gehören beispielsweise substituierte Phenole, Hydrochinone, Brenzcatechnine und aromatische Amine sowie organische Sulfide, Polysulfide, Dithiocarbamate, Phosphite und Phosphonate.

Ein erhöhter Tragekomfort kann aus der zusätzlichen Verwendung von Antistatika resultieren, die den erfindungsgemäßen Mitteln zusätzlich beigefügt werden. Antistatika vergrößern die Oberflächenleitfähigkeit und ermöglichen damit ein verbessertes Abfließen gebildeter Ladungen. Äußere Antistatika sind in der Regel Substanzen mit wenigstens einem hydrophilen Molekülliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsäureester) und schwefelhaltige (Alkylsulfonate, Alkylsulfate) Antistatika unterteilen. Lauryl- (bzw. Stearyl-) dimethylbenzylammoniumchloride eignen sich ebenfalls als Antistatika für Textilien bzw. als Zusatz zu Waschmitteln, wobei zusätzlich ein Avivageeffekt erzielt wird.

Phobier- und Imprägnierverfahren dienen der Ausrüstung von Textilien mit Substanzen, welche die Ablagerung von Schmutz verhindern oder dessen Auswaschbarkeit erleichtern. Bevorzugte Phobier- und Imprägniermittel sind perfluorierte Fettsäuren, auch in Form ihrer Aluminium- und Zirconiumsalze, organische Silicate, Silicone, Polyacrylsäureester mit perfluorierter Alkohol-Komponente oder mit perfluoriertem Acyl- oder Sulfonyl-Rest gekoppelte, polymerisierbare Verbindungen. Auch Antistatika können enthalten sein. Die schmutzabweisende Ausrüstung mit Phobier- und Imprägniermitteln wird oft als eine Pflegeleicht-Ausrüstung eingestuft. Das Eindringen der Imprägniermittel in Form von Lösungen oder Emulsionen der betreffenden Wirkstoffe kann durch Zugabe von Netzmitteln erleichtert werden, die die Oberflächenspannung herabsetzen. Ein weiteres Einsatzgebiet von Phobier- und Imprägniermitteln ist die wasserabweisende Ausrüstung von Textilwaren, Zelten, Planen, Leder usw., bei der im Gegensatz zum Wasserdichtmachen die Gewebeporen nicht verschlossen werden, der Stoff also atmungsaktiv bleibt (Hydrophobieren). Die zum Hydrophobieren verwendeten Hydrophobiermittel überziehen Textilien, Leder, Papier, Holz usw. mit einer sehr dünnen Schicht hydrophober Gruppen, wie längere Alkyl-Ketten oder Siloxan-Gruppen. Geeignete Hydrophobiermittel sind z. B. Paraffine, Wachse, Metallseifen usw. mit Zusätzen an Aluminium- oderZirconium-Salzen, quartäre Ammonium-Verbindungen mit langkettigen Alkyl-Resten, Harnstoff-Derivate, Fettsäure-modifizierte Melaminharze, Chrom-Komplexsalze, Silicone, Zinn-organische Verbindungen und Glutardialdehyd sowie perfluorierte Verbindungen. Die hydrophobierten Materialien fühlen sich nicht fettig an; dennoch perlen - ähnlich wie an gefetteten Stoffen - Wassertropfen an ihnen ab, ohne zu benetzen. So haben z. B. Silicon-imprägnierte Textilien einen weichen Griff und sind wasser- und schmutzabweisend; Flecke aus Tinte, Wein, Fruchtsäften und dergleichen sind leichter zu entfernen.

Zur Pflege der Textilien und zur Verbesserung der Textileigenschaften wie einem weicheren "Griff' (Avivage) und verringerter elektrostatischer Aufladung (erhöhter Tragekomfort) können die erfindungsgemäßen Mittel Weichspüler enthalten. Die Wirkstoffe in Weichspülformulierungen sind "Esterquats", quartäre Ammoniumverbindungen mit zwei hydrophoben Resten, wie beispielsweise das Distearyldimethylammoniumchlorid, welches jedoch wegen seiner ungenügenden biologischen Abbaubarkeit zunehmend durch quartäre Ammoniumverbindungen ersetzt wird, die in ihren hydrophoben Resten Estergruppen als Sollbruchstellen für den biologischen Abbau enthalten. Derartige "Esterquats" mit verbesserter biologischer Abbaubarkeit sind beispielsweise dadurch erhältlich, daß man Mischungen von Methyldiethanolamin und/oder Triethanolamin mit Fettsäuren verestert und die Reaktionsprodukte anschließend in an sich bekannter Weise mit Alkylierungsmitteln quaterniert. Als Appretur weiterhin geeignet ist Dimethylolethylenharnstoff.

Zur Verbesserung des Wasserabsorptionsvermögens, der Wiederbenetzbarkeit der behandelten Textilien und zur Erleichterung des Bügelns der behandelten Textilien können in den erfindungsgemäßen Mitteln beispielsweise Silikonderivate eingesetzt werden. Diese verbessern zusätzlich das Ausspülverhalten der erfindungsgemäßen Mittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H- und/oder Si-Cl-Bindungen aufweisen. Weitere bevorzugte Silikone sind die Polyalkylenoxid-modifizierten Polysiloxane, also Polysiloxane, welche beispielsweise Polyethylenglycole aufweisen sowie die Polyalkylenoxid-modifizierten Dimetylpolysiloxane.

Proteinhydrolysate sind auf Grund ihrer faserpflegenden Wirkung weitere im Rahmen der vorliegenden Erfindung bevorzugte Aktivsubstanzen aus dem Gebiet der Wasch- und Reinigungsmittel. Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte.

Die Desodorierung und Beduftung von Räumen mittels der erfindungsgemäßen Mittel erfolgt, wie eingangs erläutert, durch Einbringen dieser Mittel in einem Raum und nachfolgendes Erwärmen auf Temperaturen zwischen 30 und 150°C. Ein weiterer Gegenstand der vorliegenden Anmeldung ist daher ein Verfahren zur Desodorierung und Beduftung von Räumen, dadurch gekennzeichnet, dass ein erfindungsgemäßes Duftabgabesystem in den Raum eingebracht wird und auf Temperaturen zwischen 30 und 150°C erwärmt wird. Falls das erfindungsgemäß Duftabgabesystem in einer Umgebung eingesetzt wird, wo die Temperaturen unterhalb der Erweichungs- oder Schmelztemperatur für das eingesetzte Material des Partikel vorhanden sind, dann muss zur seiner Aktivierung eine Wärmebehandlung vor dem Gebrauch durchgeführt werden. Mögliche Einsatzorte daher sind: Toilettenräume, Fahrgastzellen, Toilettenschüsseln, Abfalleimer, Kleiderschränke.

Zur Beduftung eignen sich generell alle geschlossenen Räume, insbesondere jedoch die Innenräume von Gebäuden, Fahrzeugen oder technischen Geräten, vorzugsweise wie Textilwaschmaschinen, Trocknern oder Geschirrspülmaschinen.

Die Verwendung erfindungsgemäßer Duftabgabesystemen zur Desodorierung und Beduftung von geschlossenen oder offenen Räumen ist ein weiterer Gegenstand der vorliegenden Anmeldung.

## Patentansprüche

1. Duftabgabesystem mit einem Behälter und einer Vielzahl von im Aufnahmeraum des Behälters aufgenommenen Partikeln zur Desodorierung und/oder Beduftung von offenen bzw. geschlossenen Räumen, insbesondere Textilwaschmaschinen, Trocknern oder Geschirrspülmaschinen, wobei die Partikel ein vorzugsweise polymeres Trägermaterial sowie wenigstens einen Duftstoff aufweisen, wobei der Behälter eine Mehrzahl von Öffnungen aufweist, durch welche eine Emittierung der Duftstoffe der Partikel vom Aufnahmeraum nach außen möglich ist, **dadurch gekennzeichnet,**
**dass** der Aufnahmeraum (3) des im Wesentlichen rotationssymmetrischen Behälters (2) eine mondsichelartige Querschnittsform mit einer nach außen gewölbten Vorderwand (5) und einer nach innen gewölbten Rückwand (6) aufweist,
der Behälter (2) zweiteilig ausgebildet ist, wobei der eine Teil (2') die Rückwand (6) und der andere Teil (2") die Vorderwand (5) aufweist,
die nach innen gewölbte Rückwand (6) in ihrem mittleren Bereich (15) kegelförmig nach innen gewölbt ist,
der Aufnahmeraum (3) vollständig mit Partikeln (4) befüllt ist,
und die Partikel nicht lose im Behälter angeordnet sind.

2. Duftabgabesystem nach Anspruch 1, **dadurch gekennzeichnet,**
**dass** die beiden Endbereiche (7) der mondsichelartigen Querschnittsform des Aufnahmeraumes (3) abgerundet ausgebildet sind.

3. Duftabgabesystem nach einem der Ansprüche 1 oder 2, **dadurch gekennzeichnet,**
**dass** der die Rückwand (6) aufweisende Teil (2') des Behälters (2) einen wulstartigen Randbereich (8) aufweist, welcher mit einem stegartigen Randbereich (9) des anderen Teiles (2') verbunden ist.

4. Duftabgabesystem nach Anspruch 3, **dadurch gekennzeichnet,**
**dass** die beiden Teile (2',2") mittels einer Rastverbindung (9,10) miteinander verbunden sind.

5. Duftabgabesystem nach einem der Ansprüche 1 bis 4, **dadurch gekennzeichnet,**
**dass** die Schichtdicke der Partikel (4) im annähernd vollständig mit Partikeln (4) befüllten Aufnahmeraum (3) zwischen 10 bis 12 mm beträgt.

6. Duftabgabesystem nach einem der Ansprüche 1 bis 5, **dadurch gekennzeichnet,**
**dass** das Volumen des Aufnahmeraumes (3) von 10 bis 500 ml, zum Einsatz in Geschirrspülern vorzugsweise etwa 40 ml beträgt.

7. Duftabgabesystem nach einem der Ansprüche 1 bis 6, **dadurch gekennzeichnet, dass** der Behälter (2) außenseitig eine Aufhängeeinrichtung (16) oder sonstige Befestigungsmittel wie Klebeflächen oder dgl. aufweist.

8. Duftabgabesystem nach einem der Ansprüche 1 bis 7, **dadurch gekennzeichnet,**
**dass** im Bereich der Rückwand (6) eine Mehrzahl von schlitzförmigen Öffnungen (13,14) vorgesehen ist.

9. Duftabgabesystem nach einem der Ansprüche 1 bis 8, **dadurch gekennzeichnet, dass** das polymere Trägermaterial einen Schmelz- oder Erweichungspunkt zwischen 30 und 150°C, vorzugsweise zwischen 40 und 125°C, besonders vorzugsweise zwischen 60 und 100°C, ganz besonders bevorzugt von 70 bis 90°C und insbesondere zwischen 75 und 80°C, aufweist.

10. Duftabgabesystem nach einem der Ansprüche 1 bis 9, **dadurch gekennzeichnet, dass** das polymere Trägermaterial mindestens eine Substanz aus der Gruppe umfassend Ethylen/Vinylacetat- Copolymere, Polyethylen niederer oder hoher Dichte (LDPE, HDPE) oder Gemische derselben, Polypropylen, Polyethylen/Polypropylen-Copolymere, Polyether/Polyamid-Blockcopolymere, Styrol/Butadien-(Block-)Copolymere, Styrol/Isopren-(Block)Copolymere, Styrol/Ethylen/Butylen-Copolymere, Acrylnitril/Butadien/Styrol-Copolymere, Acrylnitril/Butadien-Copolymere, Polyetherester, Polyisobuten, Polyisopren, Ethylen/Ethylacrylat-Copolymere, Polyamide, Polycarbonat, Polyester, Polyacrylnitril, Polymethyl-methacrylat, Polyurethane, Polyvinylalkohole enthält.

11. Duftabgabesystem nach einem der Ansprüche 1 bis 10, **dadurch gekennzeichnet, dass** das polymere Trägermaterial mindestens 10 Gew.-%, vorzugsweise mindestens 30 Gew.-%, besonders bevorzugt mindestens 70 Gew.-% Ethylen/Vinylacetat-Copolymer enthält, vorzugsweise vollständig aus EthylenNinylacetat-Copolymer hergestellt ist.

12. Duftabgabesystem nach einem der Ansprüche 1 bis 11, **dadurch gekennzeichnet, dass** als polymeres Trägermaterial EthylenNinylacetat-Copolymer eingesetzt wird und dieses Copolymer 5 bis 50 Gew.-% Vinylacetat, vorzugsweise 10 bis 40 Gew.-% Vinylacetat und insbesondere 20 bis 30 Gew.-% Vinylacetat, jeweils bezogen auf das Gesamtgewicht des Copolymers, enthält.

13. Duftabgabesystem nach einem der Ansprüche 1 bis 12, **dadurch gekennzeichnet, dass** der Gewichtsanteil des/der Duftstoffe(s) 1 bis 70 Gew.-%, vorzugsweise 10 bis 60 Gew.-%, besonders bevorzugt 20 bis 50 Gew.-%, insbesondere 30 bis 40 Gew.-%, jeweils bezogen auf das Gesamtgewicht der Partikel, beträgt.

14. Duftabgabesystem nach einem der Ansprüche 1 bis 13, **dadurch gekennzeichnet, dass** die Partikel (4) einen mittleren Durchmesser von 0,5 bis 20 mm, vorzugsweise von 1 bis 10 mm und insbesondere von 3 bis 6 mm aufweisen.

15. Duftabgabesystem nach einem der Ansprüche 1 bis 14, **gekennzeichnet durch** weitere Aktivsubstanzen aus der Gruppe der Parfümträger, Farbstoffe, antimikrobiellen Wirkstoffe, Germizide, Fungizide, Antioxidantien oder Korrosionsinhibitoren, wobei diese in dem Behälter (2) und/oder dessen Aufnahmeraum (3) vorgesehen sind.

16. Duftabgabesystem nach einem der Ansprüche 1 bis 15, **dadurch gekennzeichnet, dass** der Behälter (2) aus einem wasserunlöslichen organischen oder anorganischen Material, wie Kunststoff, Keramik, Glas, Metall oder Textilien, ist.

17. Verfahren zur Desodorierung und Beduftung von geschlossenen Räumen, **gekennzeichnet durch** ein Duftabgabesystem nach einem der Ansprüche 1 bis 16, das in einen Raum eingebracht wird, wobei das Duftabgabesystem vor oder während des Gebrauches auf eine Temperatur zwischen 30 und 150°C erwärmt wird.

18. Verfahren nach Anspruch 17, **dadurch gekennzeichnet, dass** die Räume Innenräume von Gebäuden wie Toilettenräumen, Fahrzeugen oder technischen Geräten, vorzugsweise Waschmaschinen, Trocknern oder Geschirrspülmaschinen, sind.

19. Verwendung von Duftabgabesystemen nach einem der Ansprüche 1 bis 16 zur Desodorierung und Beduftung von geschlossenen oder offenen Räumen wie Toiletten, Fahrgastzellen, Kleiderschränken, Abfalleimern, Geschirrspülmaschinen, Waschmaschinen und Trocknern.

## Claims

1. Fragrance release system with a container and a plurality of particles, which are received in the receiving space of the container, for deodorising and/or scenting open or closed spaces, particularly washing machines for textiles, driers or dishwashers, wherein the particles comprise a preferably polymer carrier material as well as at least one scent, wherein the container has a plurality of openings through which emission of the scents of the particles outwardly from the receiving space is possible, **characterised in that** the receiving space (3) of the substantially rotationally symmetrical container (2) has a crescent-like cross-sectional shape with an outwardly curved front wall (5) and an inwardly curved back wall (6), the container (2) is of two-part construction, wherein one part (2') has the back wall (6) and the other part (2") the front wall (5), the inwardly curved back wall (6) is inwardly curved in cone shape in its centre region (15), the receiving space (3) is completely filled with particles (4) and the particles are not loosely arranged in the container.

2. Fragrance release system according to claim 1, **characterised in that** the two end regions (7) of the crescent-like cross-sectional form of the receiving space (3) are formed to be rounded.

3. Fragrance release system according to one of claims 1 and 2, **characterised in that** the part (2'), which has the back wall (6), of the container (2) has a bead-like edge region (8) which is connected with a web-like edge region (9) of the other part (2').

4. Fragrance release system according to claim 3, **characterised in that** the two parts (2', 2") are connected together by means of a detent connection (9, 10).

5. Fragrance release system according to one of claims 1 to 4, **characterised in that** the layer thickness of the particles (4) in the receiving space (3), which is approximately completely filled with particles (4), is between 10 to 12 millimetres.

6. Fragrance release system according to one of claims 1 to 5, **characterised in that** the volume of the receiving space (3) is from 10 to 500 millilitres, preferably approximately 40 millilitres for use in dishwashers.

7. Fragrance release system according to one of claims 1 to 6, **characterised in that** the container (2) has at the outer side a suspension device (16) or other fastening means such as adhesive surfaces or the like.

8. Fragrance release system according to one of claims 1 to 7, **characterised in that** a plurality of slot-shaped openings (13, 14) is provided in the region of the back wall (6).

9. Fragrance release system according to one of claims 1 to 8, **characterised in that** the polymer carrier material has a melting or softening point of between 30 and 150° C, preferably between 40 and 125° C, particularly preferably between 60 and 100° C, more particularly preferably from 70 to 90° C and especially between 75 and 80° C.

10. Fragrance release system according to one of claims 1 to 9, **characterised in that** the polymer carrier material contains at least one substance selected from the group comprising ethylene/vinylacetate copolymers, polyethylene of lower or higher density (LDPE, HDPE) or mixtures thereof, polypropylene, polyethylene/polypropylene copolymers, polyether/polyamide block copolymers, styrol/butadiene (block) copolymers, styrol/isoprene (block) copolymers, styrol/ethylene/butylene copolymers, acrylnitrile/butadiene/styrol copolymers, acrylnitrile/butadiene copolymers, polyetherester, polyisobutene, polyisoprene, ethylene/ethylacrylate copolymers, polyamides, polycarbonate, polyester, polyacrylnitrile, polymethyl-methacrylate, polyurethanes and polyvinlyalcohols.

11. Fragrance release system according to one of claims 1 to 10, **characterised in that** the polymer carrier material contains at least 10 weight %, preferably at least 30 weight %, particularly preferably at least 70 weight %, of ethylene/vinylacetate copolymer, preferably is made completely from ethylene/vinylacetate copolymer.

12. Fragrance release system according to one of claims 1 to 11, **characterised in that** ethylene/vinylacetate copolymer is used as polymer carrier material and this copolymer contains 5 to 50 weight % of vinylacetate, preferably 10 to 40 weight % of vinylacetate and particularly 20 to 30 weight % of vinylacetate respectively referred to the total weight of the copolymer.

13. Fragrance release system according to one of claims 1 to 12, **characterised in that** the weight proportion of the scent or scents is 1 to 70 weight %, preferably 10 to 60 weight %, particularly preferably 20 to 50 weight %, especially 30 to 40 weight %, respectively referred to the total weight of the particles.

14. Fragrance release system according to one of claims 1 to 13, **characterised in that** the particles (4) have a mean diameter of 0.5 to 20 millimetres, preferably from 1 to 10 millimetres and especially from 3 to 6 millimetres.

15. Fragrance release system according to one of claims 1 to 14, **characterised by** further active substances from the group of perfume carriers, dyes, antimicrobial active ingredients, germicides, fungicides, antioxidants or corrosion inhibitors, wherein these are provided in the container (2) and/or the receiving space (3) thereof.

16. Fragrance release system according to one of claims 1 to 15, **characterised in that** the container (2) is of a water-insoluble organic or inorganic material such as plastics material, ceramic, glass, metal or textile.

17. Method of deodorising and scenting closed spaces, **characterised by** a fragrance release system according to one of claims 1 to 16, which is introduced into a space, wherein the fragrance release system is heated to a temperature between 30 and 150° C before or during use.

18. Method according to claim 17, **characterised in that** the spaces are interior spaces of buildings such as toilets, vehicles or technical apparatus, preferably washing machines, driers or dishwashers.

19. Use of fragrance release systems according to one of claims 1 to 16 for deodorising and scenting of closed or open spaces such as toilets, passenger compartments, wardrobes, rubbish bins, dishwashers, washing machines and driers.

## Revendications

1. Système de libération de substances odoriférantes, comprenant un récipient et une multitude de particules logées dans l'espace de réception du récipient pour la désodorisation d'espaces ouverts, respectivement fermés et/ou la diffusion d'une odeur dans lesdits espaces, en particulier des lave-linge, des séchoirs ou des lave-vaisselle, les particules présentant une matière de support de préférence polymère et au moins une substance odoriférante, le récipient présentant une multitude d'orifices à travers lesquels une émission des substances odoriférantes des particules depuis l'espace de réception vers l'extérieur est possible, **caractérisé en ce que**
l'espace de réception (3) du récipient (2) essentiellement symétrique en rotation présente une section transversale en forme de croissant de lune, comportant une paroi avant (5) bombée vers l'extérieur et une paroi arrière (6) bombée vers l'intérieur ;
le récipient (2) est réalisé en deux parties, la première partie (2') présentant la paroi arrière (6) et l'autre partie (2") présentant la paroi avant (5) ;
la paroi arrière (6) bombée vers l'intérieur présente, dans sa zone médiane (15), un bombement de forme conique orienté vers l'intérieur ; l'espace de réception (3) est rempli complètement avec des particules (4) ;
et les particules sont disposées de manière non mobile dans le récipient.

2. Système de libération de substances odoriférantes selon la revendication 1, **caractérisé en ce que**
les deux zones terminales (7) de la section transversale de l'espace de réception (3) en forme de croissant de lune sont arrondies.

3. Système de libération de substances odoriférantes selon la revendication 1 ou 2, **caractérisé en ce que**
la partie (2') du récipient (2) présentant la paroi arrière (6) présente une zone marginale (8) formant un bourrelet qui est reliée à une zone marginale (9) de l'autre partie (2') en forme de traverse.

4. Système de libération de substances odoriférantes selon la revendication 3, **caractérisé en ce que**
les deux parties (2', 2") sont reliées l'une à l'autre au moyen d'une liaison d'encliquetage (9, 10).

5. Système de libération de substances odoriférantes selon l'une quelconque des revendications 1 à 4, **caractérisé en ce que**
l'épaisseur de couche des particules (4), dans l'espace de réception (3) rempli presque complètement avec des particules (4) s'élève de 10 à 12 mm.

6. Système de libération de substances odoriférantes selon l'une quelconque des revendications 1 à 5, **caractérisé en ce que**
le volume de l'espace de réception (3) s'élève de 10 à 500 ml, pour une mise en oeuvre dans des lave-vaisselle, de préférence à environ 40 ml.

7. Système de libération de substances odoriférantes selon l'une quelconque des revendications 1 à 6, **caractérisé en ce que** le récipient (2) présente, sur son côté externe, un dispositif de suspension (16) ou d'autres moyens de fixation tels que des surfaces adhésives, ou analogue.

8. Système de libération de substances odoriférantes selon l'une quelconque des revendications 1 à 7, **caractérisé en ce que**,
dans la zone de la paroi arrière (6), on prévoit une multitude d'orifices en forme de fentes (13, 14).

9. Système de libération de substances odoriférantes selon l'une quelconque des revendications 1 à 8, **caractérisé en ce que** la matière de support polymère présente un point de fusion ou de ramollissement entre 30 et 150 °C, de préférence entre 40 et 125 °C, de manière particulièrement préférée entre 60 et 100 °C, de manière tout particulièrement préférée entre 70 et 90 °C et en particulier entre 75 et 80 °C.

10. Système de libération de substances odoriférantes selon l'une quelconque des revendications 1 à 9, **caractérisé en ce que** la matière de support polymère contient au moins une substance choisie parmi le groupe comprenant des copolymères d'éthylène/acétate de vinyle, du polyéthylène faible densité ou haute densité (LDPE, HDPE) ou leurs mélanges, du polypropylène, des copolymères de polyéthylène/polypropylène, des copolymères séquencés de polyéther/polyamide, des copolymères (séquencés) de styrène-butadiène, des copolymères (séquencés) de styrène/isoprène, des copolymères de styrène/éthylène/butylène, des copolymères d'acrylonitrile/butadiène/styrène, des copolymères d'acrylonitrile/butadiène, des esters de polyéthers, du polyisobutène, du polyisoprène, des copolymères d'éthylène/acrylate d'éthyle, des polyamides, du polycarbonate, des polyesters, du polyacrylonitrile, du polyméthacrylate de méthyle, des polyuréthanes, des alcools polyvinyliques.

11. Système de libération de substances odoriférantes selon l'une quelconque des revendications 1 à 10, **caractérisé en ce que** la matière de support polymère contient un copolymère d'éthylène/acétate de vinyle à concurrence d'au moins 10 % en poids, de préférence d'au moins 30 % en poids, de manière particulièrement préférée d'au moins 70 % en poids, de préférence est préparée complètement à partir d'un copolymère d'éthylène/acétate de vinyle.

12. Système de libération de substances odoriférantes selon l'une quelconque des revendications 1 à 11, **caractérisé en ce qu'**on met en oeuvre, à titre de matière de support polymère, un copolymère d'éthylène/acétate de vinyle, ce copolymère contenant de l'acétate de vinyle à concurrence de 5 à 50 % en poids, de préférence de l'acétate de vinyle à concurrence de 10 à 40 % en poids et en particulier de l'acétate de vinyle à concurrence de 20 à 30 % en poids, chaque fois rapportés au poids total du copolymère.

13. Système de libération de substances odoriférantes selon l'une quelconque des revendications 1 à 12, **caractérisé en ce que** la fraction pondérale de la/des substance(s) odoriférante(s) s'élève de 1 à 70 % en poids, de préférence de 10 à 60 % en poids, de manière particulièrement préférée de 20 à 50 % en poids, en particulier de 30 à 40 % en poids, chaque fois rapportés au poids total des particules.

14. Système de libération de substances odoriférantes selon l'une quelconque des revendications 1 à 13, **caractérisé en ce que** les particules (4) présentent un diamètre moyen de 0,5 à 20 mm, de préférence de 1 à 10 mm et en particulier de 3 à 6 mm.

15. Système de libération de substances odoriférantes selon l'une quelconque des revendications 1 à 14 **caractérisé en ce qu'**on prévoit d'autres substances actives choisies parmi le groupe comprenant des porteurs de parfums, des colorants, des substances à activité antimicrobienne, des germicides, des fongicides, des antioxydants ou des inhibiteurs de la corrosion, ces substances étant prévues dans le récipient (2) et/ou dans l'espace de réception (3) de ce dernier.

16. Système de libération de substances odoriférantes selon l'une quelconque des revendications 1 à 15, **caractérisé en ce que** le récipient (2) est constitué d'une matière organique ou inorganique insoluble dans l'eau, telle qu'une matière synthétique, de la céramique, du verre, du métal ou des textiles.

17. Procédé pour la désodorisation d'espaces fermés et pour la diffusion d'une odeur dans lesdits espaces, **caractérisé par** un système de libération de substances odoriférantes selon l'une quelconque des revendications 1 à 16 qui est introduit dans un espace, le système de libération de substances odoriférantes étant chauffé avant ou pendant l'emploi à une température entre 30 et 150 °C.

18. Procédé selon la revendication 17, **caractérisé en ce que** les espaces sont des intérieurs de bâtiments, tels que des espaces réservés à des toilettes, des véhicules ou des appareils techniques, de préférence des lave-linge, des séchoirs ou des lave-vaisselle.

19. Utilisation de systèmes de libération de substances odoriférantes selon l'une quelconque des revendications 1 à 16 pour la désodorisation d'espaces fermés ou ouverts et pour la diffusion d'une odeur dans lesdits espaces tels que des toilettes, des habitacles, des garde-robes, des poubelles, des lave-vaisselle, des lave-linge et des séchoirs.
